# EUROPEAN PATENT APPLICATION

(11) **EP 1 329 454 A1**
(43) Date of publication of application: **23.07.2003**
(21) Application number: 01970282.8
(22) Date of filing: 28.09.2001
(51) Int. Cl.: C07D 495/04, A61K 31/519, A61K 31/5377, A61P 43/00

(54) **THIENOPYRIMIDINE COMPOUNDS AND THEIR SALTS AND PROCESS FOR PREPARATION OF BOTH**

(30) Priority: 29.09.2000 JP 2000299872
(71) Applicant: NIPPON SODA CO., LTD., Chiyoda-ku, Tokyo 100-8165 (JP)
(72) Inventor: UMEDA, Nobuhiro, Nippon Soda Co. Ltd., Odawara-shi, Kanagawa250-0280 (JP); TAKADA, Mitsumasa, Nippon Soda Co. Ltd., Odawara-shi, Kanagawa 250-0280 (JP); IKEYAMA, Seiichi, Nippon Soda Co. Ltd., Odawara-shi, Kanagawa 250-0280 (JP); ICHIKAWA, Kimiko, 1-9-30-101, Kitakatsushika-gun, Saitama 349-1101 (JP)
(74) Representative: Wibbelmann, Jobst, Dr., Dipl.-Chem.
(86) International application number: JP0108530
(87) International publication number: WO02026745

(57) **Abstract**

The invention relates to novel thienopyrimidine compounds useful as drugs having a cGMP-specific phosphodiesterase inhibiting effect or the like, specifically thieno[2,3-d]-pyrimidine compounds of the general formula (1), and a process for preparing the same: 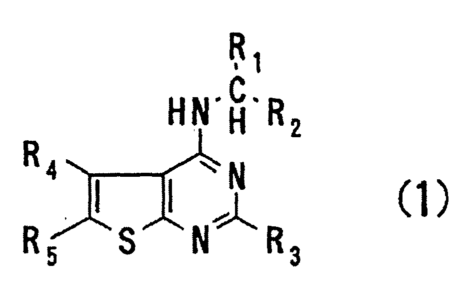 wherein R₁ is hydrogen or C₁₋₆ alkyl; R₂ is optionally substituted C₃₋₈ cycloalkyl, optionally substituted phenyl, or an optionally substituted saturated or unsaturated heterocyclic group containing one to four heteroatoms selected from among N, O and S; R₃ is an optionally substituted saturated or unsaturated heterocyclic group containing one to four heteroatoms selected from among N, O and S, (CH₂)_{K}C(=O)R₆, or CH=CHC(=O)R₆; R₄ is hydrogen, C₁₋₆ alkyl, hydroxyl, C₁₋₆ alkoxy, halogeno, C₁₋₆ haloalkyl, nitro, or cyano; and R₅ is cyano, optionally substituted phenyl, an optionally substituted saturated or unsaturated heterocyclic group containing one to four heteroatoms selected from among N, O and S, or the like.

## Description

### Technical Fields:

The present invention relates to pyridothienopyrimidine compounds useful as cGMP-specific phosphodiesterase (cGMP-PDE) inhibitors and salts thereof, and processes for the preparation of the same.

### Background Art:

cGMP is a substance playing an important role as a second messenger in the signal transduction system in vivo. Inhibitors of cGMP-PDE, which is a hydrolase of cGMP, raise cGMP levels in cells and are useful for the prevention and/or treatment of, for example, hypertension, heart failure, cardiac infarction, angina pectoris, arteriosclerosis, restenosis after PTCA (percutaneous transluminal coronary angioplasty), cardiac edema, pulmonary hypertension, renal failure, renal edema, hepatic edema, asthma, bronchitis, dementia, immunodeficiency, glaucoma or impotentia.

Compounds represented by the following formula are reported as cGMP-PDE inhibitors having thieno[2,3-d]pyrimidine skeletons in WO 98/06722, EP 728759, WO 98/17668, WO 99/28325 and WO 99/55708 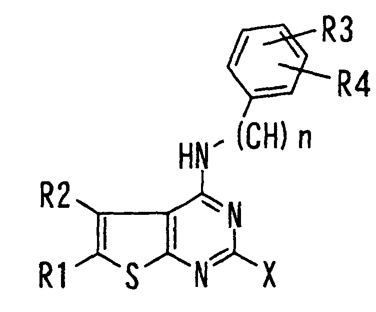 wherein, X is an optionally substituted cycloalkyl, phenyl or heterocyclic group, or alkylene or cycloalkyl substituted by a carboxylic acid, carboxylic amide or the like, and R₁ and R₂ are alkyl, nitro or halogen.

However, there are no descriptions about compounds where R₁ and R₂ in the above formula are substituents such as heterocyclic groups, carboxylic acids and carboxylic amides.

### Disclosure of the Invention:

It is an object of the present invention to provide novel thienopyrimidine compounds having cGMP-PDE inhibiting activities, and industrially advantageous processes for the preparation of them.

The present invention is directed to a thienopyrimidine compound represented by Formula (1) 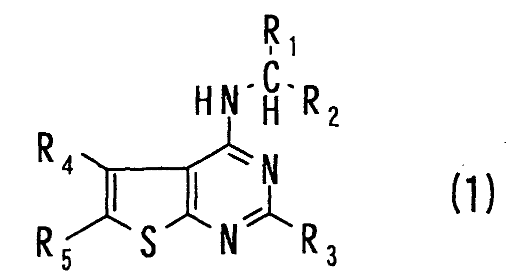 [wherein, R₁ is hydrogen or C₁₋₆ alkyl;
R₂ is C₃₋₆ cycloalkyl optionally substituted with G₁, phenyl optionally substituted with G₂, or a saturated or unsaturated heterocyclic group containing 1 to 4 heteroatoms selected from among N, O and S and optionally substituted with G₃;
R₃ is a saturated or unsaturated heterocyclic group containing 1 to 4 heteroatoms selected from among N, O and S and optionally substituted with G₃, or a group represented by Formula (CH₂)ₖC(=O)R₆ or CH=CHC(=O)R₆;
R₄ is hydrogen, C₁₋₆ alkyl, hydroxyl, C₁₋₆ alkoxy, halogen, C₁₋₆ haloalkyl, nitro or cyano;
R₅ is cyano, phenyl optionally substituted with G₂, a saturated or unsaturated heterocyclic group containing 1 to 4 heteroatoms selected from among N, O and S and optionally substituted with G₃, or a group represented by Formula (CH₂)ₖC(=O)R₆ or CH=CHC(=O)R₆;
R₆ is hydrogen, hydroxyl, C₁₋₆ alkoxy, phenoxy optionally substituted with G₂, benzyloxy optionally substituted with G2, or a group represented by Formula Nr₁r₂ or NHNr₃r₄;
r₁ and r₃ are hydrogen, or C₁₋₆ alkyl optionally substituted with hydroxyl or a ONO₂ group;
r₂ and r₄ are hydrogen, C₁₋₆ alkyl, C₃₋₈ cycloalkyl, C₁₋₆ alkoxycarbonyl, C₁₋₆ alkyl optionally substituted with a saturated or unsaturated heterocyclic group containing 1 to 4 heteroatoms selected from among N, O and S and optionally substituted with G₃, phenyl optionally substituted with G₂, benzyl optionally substituted with G₂, or a saturated or unsaturated heterocyclic group containing 1 to 4 heteroatoms selected from among N, O and S and optionally substituted with G₃;
r₁ and r₂ may join, together with N, to form a ring represented by the following formula and optionally substituted with r₅ 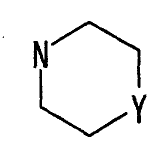 (wherein, Y is O, CH₂ or NH);
r₅ is C₁₋₆ alkyl optionally substituted with G₂, phenyl optionally substituted with G₂, benzyl optionally substituted with G₂, pyridyl optionally substituted with G₂, or -Z-Q (wherein, Z is CO, CS or SO₂, and Q is hydrogen, C₁₋₆ alkyl, C₁₋₆ alkoxy, di-C₁₋₆ alkylamino, phenyl-C₁₋₆ alkylamino, or a saturated or unsaturated heterocyclic group containing 1 to 4 heteroatoms selected from among N, O and S);
k is 0, 1 or 2;
G₁ is halogen, C₁₋₆ alkyl or C₁₋₆ alkoxy;
G₂ is halogen, C₁₋₆ alkyl, C₁₋₆ alkoxy, C₁₋₂ alkylenedioxy, C₁₋₆ alkylamino or C₁₋₆ alkylcarbamoyl;
G₃ is halogen, C₁₋₆ alkyl, C₁₋₆ haloalkyl, C₁₋₆ alkoxy or C₁₋₆ alkoxycarbonyl; and
a benzene ring, cycloalkyl group or heterocyclic ring may have two or more substituents of G₁, G₂, G₃ and r₅, which may be the same or different when two or more], and pharmaceutically acceptable salts thereof.

The present invention is directed particularly to a compound of Formula (1-2) 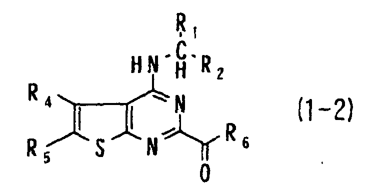 (wherein, R₁, R₂, R₄, R₅ and R₆ are as defined above).

Furthermore, it is directed to a process for the preparation of a compound of Formula (1), characterized in that a compound of Formula (2) 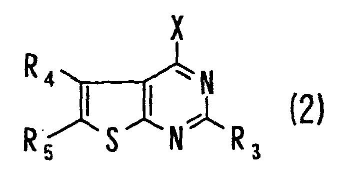 (wherein, R₃, R₄ and R₅ are as defined above and X is halogen), is reacted with a compound of Formula (3) 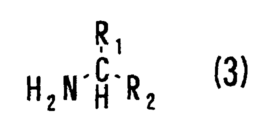 (wherein, R₁ and R₂ are as defined above).

In the compounds of the present invention, represented by Formula (1),
R₁ is hydrogen; or C₁₋₆ alkyl such as methyl, ethyl, propyl, isopropyl, butyl, isobutyl, sec-butyl or tert-butyl.
R₂ is C₃₋₈ cycloalkyl (optionally substituted at arbitrary positions with halogen such as fluorine, chlorine or bromine; C₁₋₆ alkyl such as methyl or ethyl; or C₁₋₆ alkoxy such as methoxy or ethoxy); phenyl (optionally substituted at arbitrary positions with halogen such as fluorine, chlorine or bromine; C₁₋₆ alkyl such as methyl or ethyl; C₁₋₆ alkoxy such as methoxy or ethoxy; or C₁₋₂ alkylenedioxy such as methylenedioxy or ethylenedioxy), or a saturated or unsaturated heterocyclic group containing 1 to 4 heteroatoms selected from among N, O and S (optionally substituted at arbitrary positions with halogen such as fluorine, chlorine or bromine; C₁₋₆ alkyl such as methyl or ethyl; C₁₋₆ haloalkyl such as chloromethyl, dichloromethyl, trichloromethyl, fluoromethyl, difluoromethyl or trifluoromethyl; C₁₋₆ alkoxy such as methoxy or ethoxy; or C₁₋₆ alkoxycarbonyl such as methoxycarbonyl or ethoxycarbonyl) such as furyl, thienyl, pyrrolyl, imidazolyl, pyrazolyl, oxazolyl, isoxazolyl, thiazolyl, isothiazolyl, imidazolyl, triazolyl, oxadiazolyl, pyridyl, pyrimidyl or pyridazinyl.
R₃ is a saturated or unsaturated heterocyclic group containing 1 to 4 heteroatoms selected from among N, O and S (optionally substituted at arbitrary positions with halogen such as fluorine, chlorine or bromine; C₁₋₆ alkyl such as methyl or ethyl; C₁₋₆ haloalkyl such as chloromethyl, dichloromethyl, trichloromethyl, fluoromethyl, difluoromethyl or trifluoromethyl; C₁₋₆ alkoxy such as methoxy or ethoxy; or C₁₋₆ alkoxycarbonyl such as methoxycarbonyl or ethoxycarbonyl) such as furyl, thienyl, pyrrolyl, imidazolyl, pyrazolyl, oxazolyl, isoxazolyl, thiazolyl, isothiazolyl, imidazolyl, triazolyl, oxadiazolyl, pyridyl, pyrimidyl or pyridazinyl, or a group represented by Formula (CH₂)ₖC(=O)R₆ or CH=CHC(=O)R₆.
R₄ is hydrogen; C₁₋₆ alkyl such as methyl, ethyl, propyl, isopropyl, butyl, isobutyl, sec-butyl or tert-butyl; hydroxyl; C₁₋₆ alkoxy such as methoxy or ethoxy; halogen such as fluorine, chlorine or bromine; C₁₋₆ alkyl such as methyl or ethyl; C₁₋₆ haloalkyl such as chloromethyl, dichloromethyl, trichloromethyl, fluoromethyl, difluoromethyl or trifluoromethyl; nitro or cyano.
R₅ is cyano; phenyl (optionally substituted at arbitrary positions with halogen such as fluorine, chlorine or bromine; C₁₋₆ alkyl such as methyl or ethyl; C₁₋₆ alkoxy such as methoxy or ethoxy; C₁₋₆ alkoxycarbonyl such as methoxycarbonyl or ethoxycarbonyl; or C₁₋₆ haloalkyl such as chloromethyl, dichloromethyl, trichloromethyl, fluoromethyl, difluoromethyl or trifluoromethyl); or a saturated or unsaturated heterocyclic group containing 1 to 4 heteroatoms selected from among N, O and S (optionally substituted at arbitrary positions with halogen such as fluorine, chlorine or bromine; C₁₋₆ alkyl such as methyl or ethyl; C₁₋₆ haloalkyl such as chloromethyl, dichloromethyl, trichloromethyl, fluoromethyl, difluoromethyl or trifluoromethyl; C₁₋₆ alkoxy such as methoxy or ethoxy; or C₁₋₆ alkoxycarbonyl such as methoxycarbonyl or ethoxycarbonyl), such as furyl, thienyl, pyrrolyl, imidazolyl, pyrazolyl, oxazolyl, isoxazolyl, thiazolyl, isothiazolyl, imidazolyl, triazolyl, oxadiazolyl, pyridyl, pyrimidyl or pyridazinyl; or a group represented by Formula (CH₂)ₖC(=O)R₆ or CH=CHC(=O)R₆.
R₆ is hydroxyl; C₁₋₆ alkoxy such as methoxy or ethoxy; phenoxy (optionally substituted at arbitrary positions with halogen such as fluorine, chlorine or bromine; C₁₋₆ alkyl such as methyl or ethyl; C₁₋₆ alkoxy such as methoxy or ethoxy; C₁₋₆ alkoxycarbonyl such as methoxycarbonyl or ethoxycarbonyl; or C₁₋₆ haloalkyl such as chloromethyl, dichloromethyl, trichloromethyl, fluoromethyl, difluoromethyl or trifluoromethyl); benzyloxy (optionally substituted at arbitrary positions with halogen such as fluorine, chlorine or bromine; C₁₋₆ alkyl such as methyl or ethyl; C₁₋₆ alkoxy such as methoxy or ethoxy; C₁₋₆ alkoxycarbonyl such as methoxycarbonyl or ethoxycarbonyl; or C₁₋₆ haloalkyl such as chloromethyl, dichloromethyl, trichloromethyl, fluoromethyl, difluoromethyl or trifluoromethyl), or a group represented by Formula Nr₁r₂ or NHNr₃r₄.
r₁ and r₃ are hydrogen or C₁₋₆ alkyl (optionally substituted with hydroxyl or a ONO₂ group) such as methyl or ethyl.
r₂ and r₄ are hydrogen; C₃₋₈ cycloalkyl such as cyclopropyl, cyclobutyl, cyclopentyl or cyclohexyl; C₁₋₆ alkyl such as methyl, ethyl, propyl, isopropyl or butyl; C₁₋₆ alkoxycarbonyl C₁₋₆ alkyl such as methoxycarbonylmethyl, ethoxycarbonylmethyl, methoxycarbonylethyl or ethoxycarbonylethyl; C₁₋₆ alkyl optionally substituted with a saturated or unsaturated heterocyclic group containing 1 to 4 heteroatoms selected from among N, O and S (which is optionally substituted at arbitrary positions with halogen such as fluorine, chlorine or bromine; C₁₋₆ alkyl such as methyl or ethyl; C₁₋₆ haloalkyl such as chloromethyl, dichloromethyl, trichloromethyl, fluoromethyl, difluoromethyl or trifluoromethyl; C₁₋₆ alkoxy such as methoxy or ethoxy; or C₁₋₆ alkoxycarbonyl such as methoxycarbonyl or ethoxycarbonyl), such as furyl, thienyl, pyrrolyl, imidazolyl, pyrazolyl, oxazolyl, isoxazolyl, thiazolyl, isothiazolyl, imidazolyl, triazolyl, oxadiazolyl, pyridyl, pyrimidyl or pyridazinyl; phenyl (optionally substituted at arbitrary positions with halogen such as fluorine, chlorine or bromine; C₁₋₆ alkyl such as methyl or ethyl; C₁₋₆ alkoxy such as methoxy or ethoxy; amino; or C₁₋₆ alkoxycarbonylamino such as tert-butoxycarbonylamino); benzyl (optionally substituted at arbitrary positions with halogen such as fluorine, chlorine or bromine; C₁₋₆ alkyl such as methyl or ethyl; or C₁₋₆ alkoxy such as methoxy or ethoxy); or a saturated or unsaturated heterocyclic group containing 1 to 4 heteroatoms selected from among N, O and S (optionally substituted at arbitrary positions with halogen such as fluorine, chlorine or bromine; C₁₋₆ alkyl such as methyl or ethyl; C₁₋₆ haloalkyl such as chloromethyl, dichloromethyl, trichloromethyl, fluoromethyl, difluoromethyl or trifluoromethyl; C₁₋₆ alkoxy such as methoxy or ethoxy; or C₁₋₆ alkoxycarbonyl such as methoxycarbonyl or ethoxycarbonyl), such as furyl, thienyl, pyrrolyl, imidazolyl, pyrazolyl, oxazolyl, isoxazolyl, thiazolyl, isothiazolyl, imidazolyl, triazolyl, oxadiazolyl, pyridyl, pyrimidyl or pyridazinyl.
r₁ and r₂ may join, together with N, to form a morpholino, piperidino or piperazinyl group.
r₅, a substituent on these, is C₁₋₆ alkyl (optionally substituted with C₁₋₆ alkylamino such as methylamino, ethylamino, dimethylamino or diethylamino; or C₁₋₆ alkylcarbamoyl such as methylcarbamoyl, dimethylcarbamoyl or diethylcarbamoyl) such as methyl, ethyl, propyl, isopropyl, butyl, isobutyl, sec-butyl or tert-butyl; phenyl (optionally substituted with fluorine, chlorine, methyl, ethyl, methoxy or ethoxy); benzyl (optionally substituted with fluorine, chlorine, methyl, ethyl, methoxy or ethoxy); pyridyl (optionally substituted with fluorine, chlorine, methyl, ethyl, methoxy or ethoxy); or a group represented by Formula -Z-Q (wherein, Z is CO, CS or SO₂, and Q is hydrogen, C₁₋₆ alkyl such as methyl, ethyl, propyl, isopropyl, butyl, isobutyl, sec-butyl or tert-butyl; C₁₋₆ alkoxy such as methoxy, ethoxy, propoxy, isopropoxy, butoxy, isobutoxy, sec-butoxy or tert-butoxy, di-C₁₋₆ alkylamino such as dimethylamino, diethylamino or methylethylamino; phenyl-C₁₋₆ alkylamino such as phenylmethylamino or phenylethylamino; or a saturated or unsaturated heterocyclic group containing 1 to 4 heteroatoms selected from among N, O and S (optionally substituted with C₁₋₆ alkyl such as methyl, ethyl or propyl), such as pyridyl, pyrrolidinyl, tetrahydrofuranyl, morpholino, piperidino or piperazinyl).
k is 0, 1 or 2.

The substituents may be the same or different, if the said phenyl, benzyl and heterocyclic groups have two or more of them.

Examples of pharmaceutically acceptable salts include salts of inorganic acids such as hydrochloric acid, sulfuric acid, nitric acid and phosphoric acid and of organic acids such as acetic acid, propionic acid, lactic acid, succinic acid, tartaric acid, citric acid, benzoic acid, salicylic acid, nicotinic acid and heptagluconic acid, of compounds of Formula (1).

The compounds of the present invention may have asymmetric carbons, depending on groups represented by R₁, R₂, R₃, R₄ and R₅. The present invention covers optically active compounds as well as racemic ones.

Processes of the present invention and processes for the preparation of novel compounds to be used as intermediates or others are described below.

### Process 1

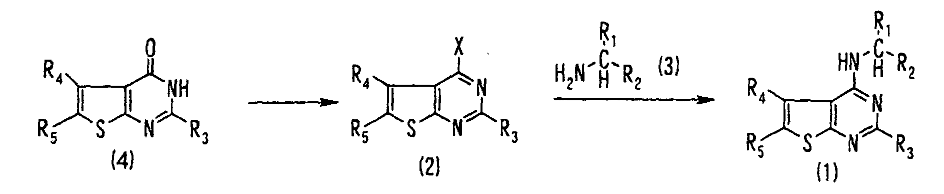

(In the above equation, R₁, R₂, R₃, R₄ and R₅ are as defined above and X is halogen.)

Target compound (1) is obtained by a substitution reaction of Compounds (2) and (3) in a solvent according to an ordinary method.

There are no particular restrictions on solvents used, if inert to the reaction. Examples of solvents include ethers such as diethyl ether, tetrahydrofuran (THF) and 1,4-dioxane; aromatic hydrocarbons such as benzene, toluene and xylene; halogenated hydrocarbons such as dichloromethane, chloroform and 1,2-dichloroethane; and pyridine, acetonitrile, dimethylformamide (DMF) and dimethyl sulfoxide (DMSO).

Reaction temperature is about -15°C to the boiling point of a solvent used, preferably 0 to 80°C.

Halogenation of thienopyrimidone of Compound (4) gives Compound (2). Examples of halogen X include chlorine and bromine.

The halogenation reaction is carried out by an ordinary method. For example, in the case of chlorination, a method is applied of using phosphorus oxychloride, phosphorus pentachloride, thionyl chloride or the like as a chlorinating agent.

There are no particular restrictions on solvents used, if inert to the reaction. Examples of solvents include aromatic hydrocarbons such as benzene, toluene and xylene; halogenated hydrocarbons such as dichloromethane, chloroform and 1,2-dichloroethane; acetonitrile, DMF and DMSO.

Reaction temperature is about -15°C to the boiling point of a solvent used, preferably 20°C to the boiling point of a solvent.

A starting material, thienopyrimidone of Compound (2), can be prepared according to known methods disclosed in papers, for example, J. Het. Chem., 21, 375-380 (1984) or Indian J. Chem., 28B (12) 1039-1047 (1989).

A starting material, Compound (3), is also prepared according to known methods disclosed in papers, for example, J. Med. Chem., 41, 3367-3372 (1998).

### Process 2

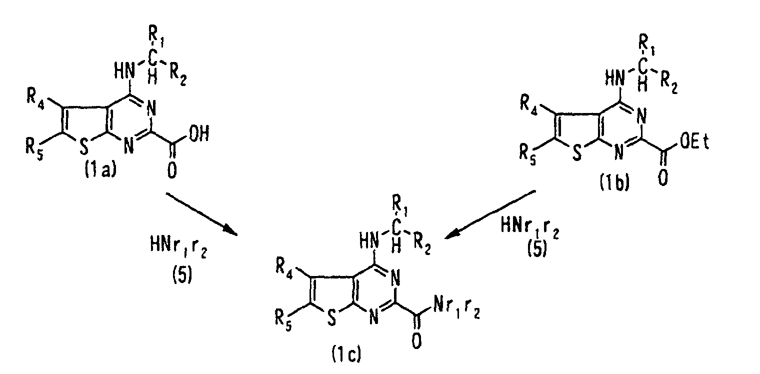

(In the above reaction scheme, R₁, R₂, R₄, R₅, r₁ and r₂ are as defined above.)

Dehydration condensation of Compound (la) and Compound (5) in a solvent by an ordinary method gives Compound (1c).

There are no particular restrictions on the dehydration condensation reaction, if an ordinary method is applied. A method of using a condensing agent is preferred.

Examples of condensing agents include 1,3-dicyclohexylcarbodiimide, 1-(3-dimethylaminopropyl)-3-ethylcarbodiimide and 2-ethoxy-1-ethoxycarbonyl-1,2-dihydroquinoline.

This reaction proceeds more promptly if N-hydroxysuccinimide, 1-hydroxybenzotriazole or 3,4-dihydro-3-hydroxy-4-oxo-1,2,3-benzotriazine coexists.

There are no particular restrictions on solvents used, if inert to the reaction. Examples of solvents include ethers such as diethyl ether, THF and 1,4-dioxane; aromatic hydrocarbons such as benzene, toluene and xylene; halogenated hydrocarbons such as dichloromethane, chloroform and 1,2-dichloroethane; pyridine, acetonitrile, DMF and DMSO.

Reaction temperature is about -15°C to the boiling point of a solvent used, preferably 0 to 80°C.

An amide derivative of Formula (1c) can also be prepared from Compound (1b).

The reaction is carried out in a solvent including an alcohol such as methanol, ethanol or propanol; halogenated hydrocarbon such as dichloromethane, chloroform or 1,2-dichloroethane; acetonitrile, DMF or DMSO, at a reaction temperature from -15 to 200°C, preferably 0 to 150°C.

### Process 3

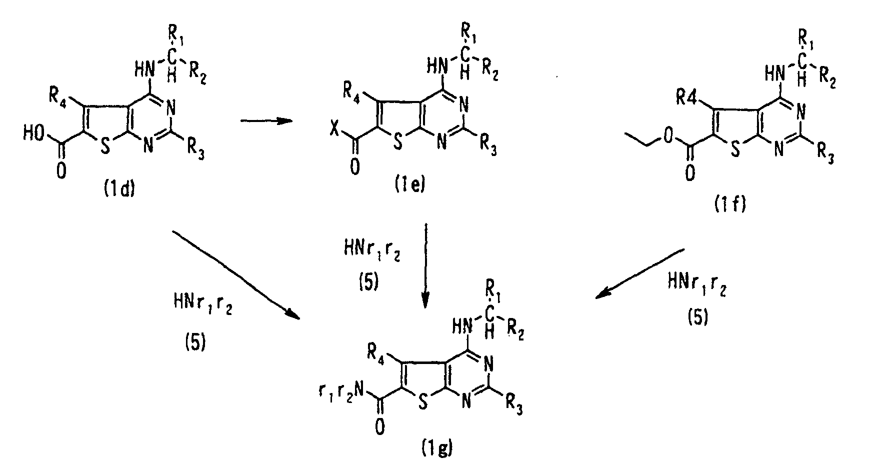

(In the above reaction scheme, R₁, R₂, R₃, R₄, r₁ and r₂ are as defined above, and X is halogen.)

Dehydration condensation of Compound (1d) and Compound (5) in a solvent by an ordinary method gives Compound (1g).

There are no particular restrictions on the dehydration condensation reaction, if an ordinary method is applied. A method of using a condensing agent is preferred.

Examples of condensing agents include 1,3-dicyclohexylcarbodiimide, 1-(3-dimethylaminopropyl)-3-ethylcarbodiimide and 2-ethoxy-1-ethoxycarbonyl-1,2-dihydroquinoline.

This reaction proceeds more promptly ifN-hydroxysuccinimide, 1-hydroxybenzotriazole or 3,4-dihydro-3-hydroxy-4-oxo-1,2,3-benzotriazine coexists.

There are no particular restrictions on solvents used, if inert to the reaction. Examples of solvents include ethers such as diethyl ether, THF and 1,4-dioxane; aromatic hydrocarbons such as benzene, toluene and xylene; halogenated hydrocarbons such as dichloromethane, chloroform and 1,2-dichloroethane; pyridine, acetonitrile, DMF and DMSO.

Reaction temperature is about -15°C to the boiling point of a solvent used, preferably 0 to 80°C.

Compound (1g) can also be prepared by that an acid halide (1e) synthesized from Compound (1d) is reacted with Compound (5).

There are no particular restrictions on the synthesis of the acid halide, if an ordinary method is applied. For example, in the case of chlorination, the reaction is carried out using phosphorus oxychloride, phosphorus pentachloride, thionyl chloride or the like as a chlorinating agent.

There are no particular restrictions on solvents used, if inert to the reaction. The reaction can be carried out without using a solvent. Examples of solvents, if used, include aromatic hydrocarbons such as benzene, toluene and xylene; and halogenated hydrocarbons such as dichloromethane, chloroform and 1,2-dichloromethane.

Reaction temperature is about 0°C to the boiling point of a solvent used, preferably 0 to 80°C.

There are no particular restrictions on the reaction of an acid chloride (1e) and Compound (5), if an ordinary method is applied. The reaction of (1e) is carried out using an excessive amount of Compound (5) or Compound (1e) is reacted with Compound (5) in the presence of an organic or inorganic base.

There are no particular restrictions on solvents used, if inert to the reaction. The reaction can be carried out without using a solvent. Examples of solvents, if used, include ethers such as diethyl ether, THF and 1,4-dioxane; aromatic hydrocarbons such as benzene, toluene and xylene; and halogenated hydrocarbons such as dichloromethane, chloroform and 1,2-dichloroethane.

Reaction temperature is about 0°C to the boiling point of a solvent used, preferably 0 to 30°C.

An amide derivative of Formula (1g) can also be prepared from Compound (1f).

The reaction is carried out in a solvent including an alcohol such as methanol, ethanol or propanol; halogenated hydrocarbon such as dichloromethane, chloroform or 1,2-dichloroethane; acetonitrile, DMF or DMSO, at a reaction temperature from -15 to 200°C, preferably 0 to 150°C.

### Process 4

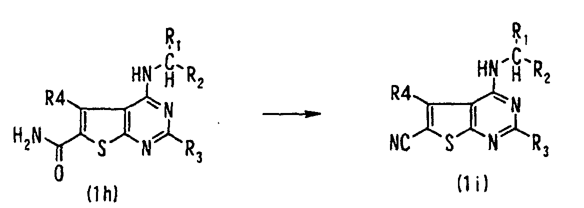

(In the above equation, R₁, R₂, R₃ and R₄ are as defined above.)

A dehydration reaction of Compound (1h) gives Compound (1i).

There are no particular restrictions on the dehydration reaction if an ordinary method is applied. The reaction is carried out, for example, using phosphorus oxychloride, phosphorus pentachloride or the like.

There are no particular restrictions on solvents used, if inert to the reaction. The reaction can be carried out without using a solvent. Examples of solvents, if used, include ethers such as diethyl ether, tetrahydrofuran (THF) and 1,4-dioxane; aromatic hydrocarbons such as benzene, toluene and xylene; halogenated hydrocarbons such as dichloromethane, chloroform and 1,2-dichloroethane; acetonitrile, DMF and DMSO.

Reaction temperature is about -15°C to the boiling point of a solvent used, preferably 0 to 80°C.

### Process 5

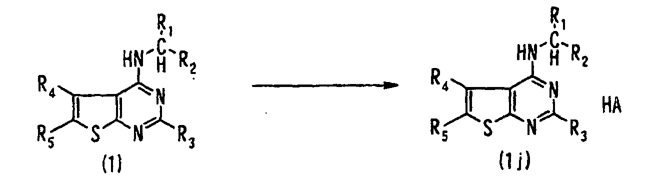

(In the above equation, R₁, R₂, R₃, R₄ and R₅ are as defined above and HA is an acid.)

Target Compound (1j) can be prepared by reacting Compound (1) with an acid.

Examples of acids used include inorganic acids such as hydrochloric acid, sulfuric acid, nitric acid and phosphoric acid; and organic acids such as acetic acid, propionic acid, lactic acid, succinic acid, tartaric acid, citric acid, benzoic acid, salicylic acid, nicotinic acid and heptagluconic acid.

There are no particular restrictions on solvents used, if inert to the reaction. Examples of solvents include ethers such as diethyl ether, THF and 1,4-dioxane; aromatic hydrocarbons such as benzene, toluene and xylene; halogenated hydrocarbons such as dichloromethane, chloroform and 1,2-dichloroethane; acetonitrile, DMF and DMSO.

Reaction temperature is about -15°C to the boiling point of a solvent used, preferably 0 to 30°C.

Compound (1) of the present invention may have asymmetric carbons in Formula (1) so that optical isomers exist. It goes without saying that the present invention covers these isomers.

In the present invention, usual post-treatments give target compounds after the completion of the reactions.

The structures of the compounds of the present invention were determined by IR, NMR, MS and other means.

### Best Forms to Implement the Invention

The present invention is described in more detail in reference to Examples.

### Example 1

Preparation of 4-[(3-chloro-4-methoxy)benzylamino]-6-ethoxycarbonyl-5-methyl-2-(3-pyridyl)-thieno[2,3-d]pyrimidine

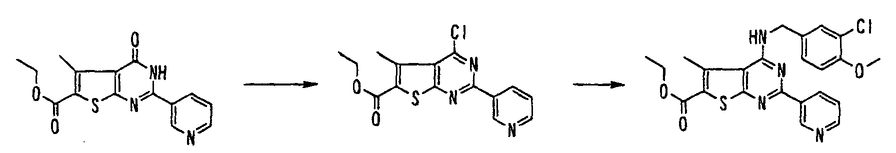

7.3g of 4-oxo-6-ethoxycarbonyl-5-methyl-2-(3-pyridyl)-thieno[2,3-d]pyrimidine was added to 60 ml of phosphorus oxychloride, and stirred at 80 to 100°C for 3 hours. After phosphorus oxychloride was distilled off under reduced pressure, 100 ml of water was added to the resulting concentrate. The reaction solution was made alkaline with an aqueous saturated solution of sodium hydrogen carbonate while cooling, and extracted with chloroform. The organic layer was washed with saturated salt water and dried over anhydrous magnesium sulfate. Magnesium sulfate was filtered off. The filtrate was concentrated under reduced pressure to give 7.4g of 4-chloro-6-ethoxycarbonyl-5-methyl-2-(3-pyridyl)-thieno[2,3-d]pyrimidine.

To 7.4g of 4-chloro-6-ethoxycarbonyl-5-methyl-2-(3-pyridyl)-thieno[2,3-d]pyrimidine were added 60 ml of DMSO, 4.2g of 3-chloro-4-methoxybenzylamine and 2.9g of triethylamine, and heated with stirring at 80°C for 3 hours. The reaction solution was poured into water. The deposited crystals were separated by filtration and dried to give 5.4g of the title compound. m.p. 216-218°C

### Example 2

Preparation of 4-[(3-chloro-4-methoxy)benzylamino]-5-methyl-2-(3-pyridyl)-thieno[2,3-d]pyrimidine-6-carboxylic acid

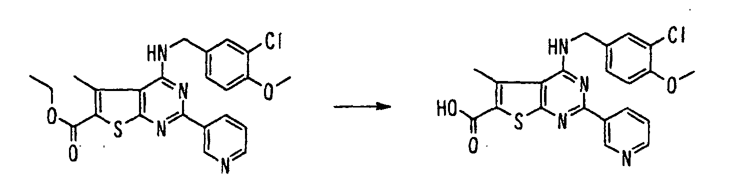

To 3.1 g of 4-[(3-chloro-4-methoxy)benzylamino]-6-ethoxycarbonyl-5-methyl-2-(3-pyridyl)-thieno[2,3-d]pyrimidine were added 60 ml of ethanol, 30 ml of water and 1.5g of sodium hydroxide, and heated at reflux for 2 hours. The reaction solution was concentrated under reduced pressure and poured into water. The resulting solution was made pH 6 with 2N hydrochloric acid. The deposited crystals were separated by filtration and dried to give 2.9g of the title compound. m.p. 185-186°C

### Example 3

Preparation of 4-[(3-chloro-4-methoxy)benzylamino]-5-methyl-2-(3-pyridyl)-thieno[2,3-d]pyrimidine-6-N-phenylcarboxamide

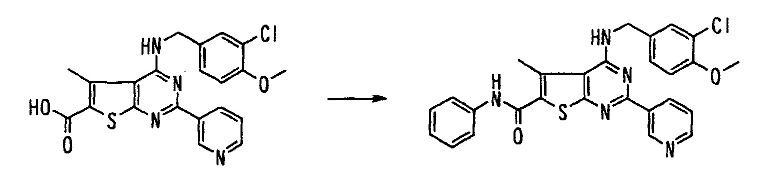

To 0.3g of 4-[(3-chloro-4-methoxy)benzylamino]-5-methyl-2-(3-pyridyl)-thieno[2,3-d]pyrimidine-6-carboxylic acid were added 0.16g of 1-(3-dimethylaminopropyl)-3-ethylcarbodiimide hydrochloride, 0.11 g of 1-hydroxybenzotriazole hydrochloride, 0.1 of triethylamine and 0.07g of aniline, dissolved in 20ml of DMF and stirred at room temperature for 20 hours. The reaction solution was poured into water. The deposited crystals were separated by filtration, washed with water and ether, and dried to give 0.23g of the title compound. m.p. 212-213°C

### Example 4

Preparation of 4-[(3-chloro-4-methoxy)benzylamino]-5-methyl-2-(3-pyridyl)-thieno[2,3-d]pyrimidine-6-carboxamide

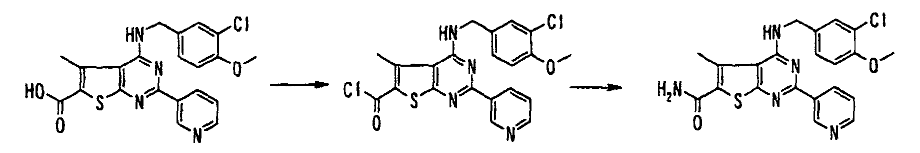

To 0.63g of 4-[(3-chloro-4-methoxy)benzylamino]-5-methyl-2-(3-pyridyl)-thieno[2,3-d]pyrimidine-6-carboxylic acid were added 8 ml of thionyl chloride and a drop of pyridine, and heated at reflux for 2 hours. The reaction solution was concentrated to give 0.7g of 4-[(3-chloro-4-methoxy)benzylamino]-5-methyl-2-(3-pyridyl)-thieno[2,3-d]pyrimidine-6-carboxylic acid chloride.

To 0.7g of 4-[(3-chloro-4-methoxy)benzylamino]-5-methyl-2-(3-pyridyl)-thieno[2,3-d]pyrimidine-6-carboxylic acid chloride were added 10 ml of tetrahydrofuran and 10 ml of aqueous ammonia, and stirred at room temperature for 2 hours. The reaction solution was poured into water and extracted with ethyl acetate. The organic layer was washed with saturated salt water and dried over anhydrous magnesium sulfate. Magnesium sulfate was filtered off. The filtrate was concentrated under reduced pressure. The obtained crystals were washed with ether to give 0.6g of the title compound. m.p. 220-222°C

### Example 5

Preparation of 4-[(3-chloro-4-methoxy)benzylamino]-6-cyano-5-methyl-2-(3-pyridyl)-thieno[2,3-d]pyrimidine

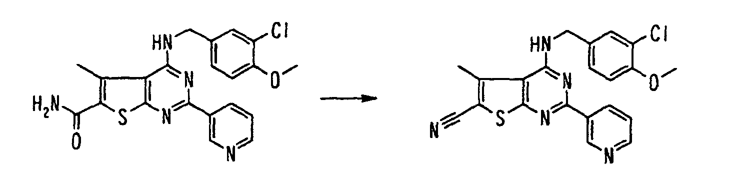

To 0.45g of 4-[(3-chloro-4-methoxy)benzylamino]-5-methyl-2-(3-pyridyl)-thieno[2,3-d]pyrimidine-6-carboxamide was added 5 ml of phosphorus oxychloride, and heated at reflux for 30 minutes. The reaction solution was poured into water, neutralized with a 2N aqueous solution of sodium hydroxide and extracted with ethyl acetate. The organic layer was washed with saturated salt water and dried over anhydrous magnesium sulfate. Magnesium sulfate was filtered off. The filtrate was concentrated under reduced pressure. The obtained crystals were washed with ether to give 0.2g of the title compound. m.p. 182-185°C

### Example 6

Preparation of 4-[(3-chloro-4-methoxy)benzylamino]-2-ethoxycarbonyl-5-methyl-6-(3-pyridyl)-thieno[2,3-d]pyrimidine

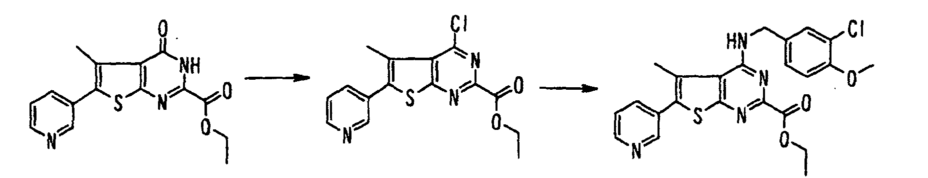

4.5g of 4-oxo-2-ethoxycarbonyl-5-methyl-6-(3-pyridyl)-thieno[2,3-d]pyrimidine was added to 40 ml of phosphorus oxychloride, and stirred at 80 to 100°C for 3 hours. After phosphorus oxychloride was distilled off under reduced pressure, 80 ml of water was added to the resulting concentrate. The reaction solution was made alkaline with an aqueous saturated solution of sodium hydrogen carbonate while cooling, and extracted with chloroform. The organic layer was washed with saturated salt water and dried over anhydrous magnesium sulfate. After magnesium sulfate was filtered off, the filtrate was concentrated under reduced pressure to give 4.1g of 4-chloro-2-ethoxycarbonyl-5-methyl-6-(3-pyridyl)-thieno[2,3-d]pyrimidine.

To 4.1 g of 4-chloro-2-ethoxycarbonyl-5-methyl-6-(3-pyridyl)-thieno[2,3-d]pyrimidine were added 30 ml of DMSO, 2.3g of 3-chloro-4-methoxybenzylamine and 1.6g of triethylamine, and heated with stirring at 80°C for 3 hours. The reaction solution was poured into water. The deposited crystals were separated by filtration and dried to give 5.1g of the title compound. m.p. 172-174°C

### Example 7

Preparation of 4-[(3-chloro-4-methoxy)benzylamino]-5-methyl-6-(3-pyridyl)-thieno[2,3-d]pyrimidine-2-carboxylic acid

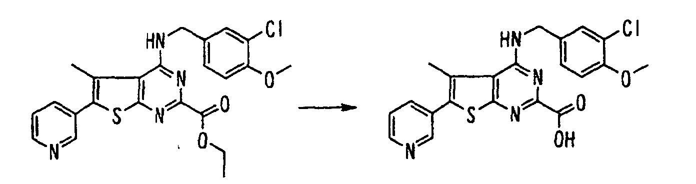

To 2.5g of 4-[(3-chloro-4-methoxy)benzylamino]-2-ethoxycarbonyl-5-methyl-6-(3-pyridyl)-thieno[2,3-d]pyrimidine were added 20 ml of ethanol, 10 ml of water and 0.43g of sodium hydroxide, and heated at reflux for 2 hours. The reaction solution was concentrated under reduced pressure and poured into water. The resulting solution was made pH 4 with 2N hydrochloric acid. The deposited crystals were separated by filtration and dried to give 2.3g of the title compound. m.p. 222-224°C

### Example 8

Preparation of 4-[(3-chloro-4-methoxy)benzylamino]-5-methyl-6-(3-pyridyl)-thieno[2,3-d]pyrimidine-2-N-phenylcarboxamide

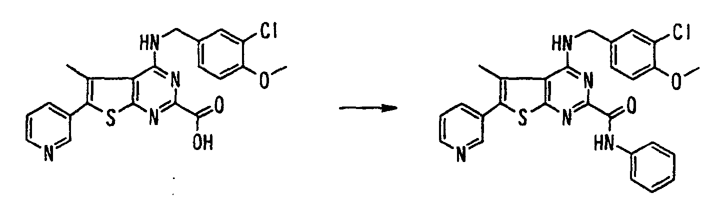

To 0.3g of 4-[(3-chloro-4-methoxy)benzylamino]-5-methyl-6-(3-pyridyl)-thieno[2,3-d]pyrimidine-2-carboxylic acid were added 0.16g of 1-(3-dimethylaminopropyl)-3-ethylcarbodiimide hydrochloride, 0.11g of 1-hydroxybenzotriazole hydrochloride, 0.1g of triethylamine and 0.07g of aniline, dissolved in 20ml of DMF and stirred at room temperattue for 20 hours. The reaction solution was poured into water. The deposited crystals were separated by filtration, washed with water and ether, and dried to give 0.32g of the title compound. m.p. 190-193°C

### Example 9

Preparation of 4-[(3-chloro-4-methoxy)benzylamino]-5-methyl-2-(3-pyridyl)-6-(4-pyridyl)-thieno[2,3-d]pyrimidine

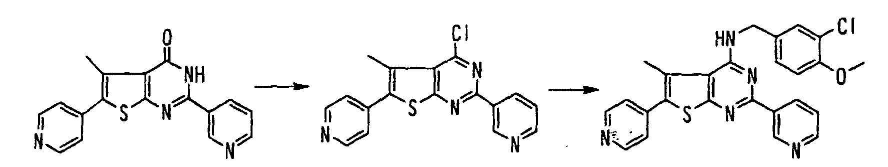

0.38g of 4-oxo-5-methyl-2-(3-pyridyl)-6-(4-pyridyl)-thieno[2,3-d]pyrimidine was added to 5 ml of phosphorus oxychloride, and stirred at 80 to 100°C for 3 hours. After phosphorus oxychloride was distilled off under reduced pressure, 30 ml of water was added to the resulting concentrate. The reaction solution was made alkaline with an aqueous saturated solution of sodium hydrogen carbonate while cooling, and extracted with chloroform. The organic layer was washed with saturated salt water and dried over anhydrous magnesium sulfate. After magnesium sulfate was filtered off, the filtrate was concentrated under reduced pressure to give 0.25g of 4-chloro-5-methyl-2-(3-pyridyl)-6-(4-pyridyl)-thieno[2,3-d]pyrimidine.

To 0.25g of 4-chloro-5-methyl-2-(3-pyridyl)-6-(4-pyridyl)-thieno[2,3-d]pyrimidine were added 5 ml of DMSO, 0.14g of 3-chloro-4-methoxybenzylamine and 0.1g of triethylamine, and heated with stirring at 80°C for 3 hours. The reaction solution was poured into water. The deposited crystals were separated by filtration and dried to give 0.32g of the title compound. m.p. 234-236°C

### Example 10

Preparation of 4-[(3-chloro-4-methoxy)benzylamino]-5-methyl-6-(3-pyridyl)-thieno[2,3-d]pyrimidine-2-N-phenylcarboxamide hydrochloride

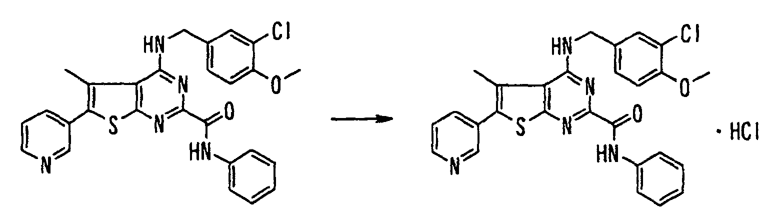

0.22g of 4-[(3-chloro-4-methoxy)benzylamino]-5-methyl-6-(3-pyridyl)-thieno[2,3-d]pyrimidine-2- N-phenylcarboxamide was dissolved in 15 ml of chloroform, and, while cooling with ice, 5 ml of ethanol saturated with hydrogen chloride gas was added. The resulting solution was stirred at room temperature for an hour. The solvent was distilled off under reduced pressure. The obtained crude crystals were washed with ether and dried to give 0.17g of the title compound. m.p. 170-175°C

Representative examples of the compounds of the present invention, including those of the above examples, are shown in Table 1. The NMR data of oily products are shown in Table 2.

Abbreviations in the table have the following meanings:
Me: methyl, Et: ethyl

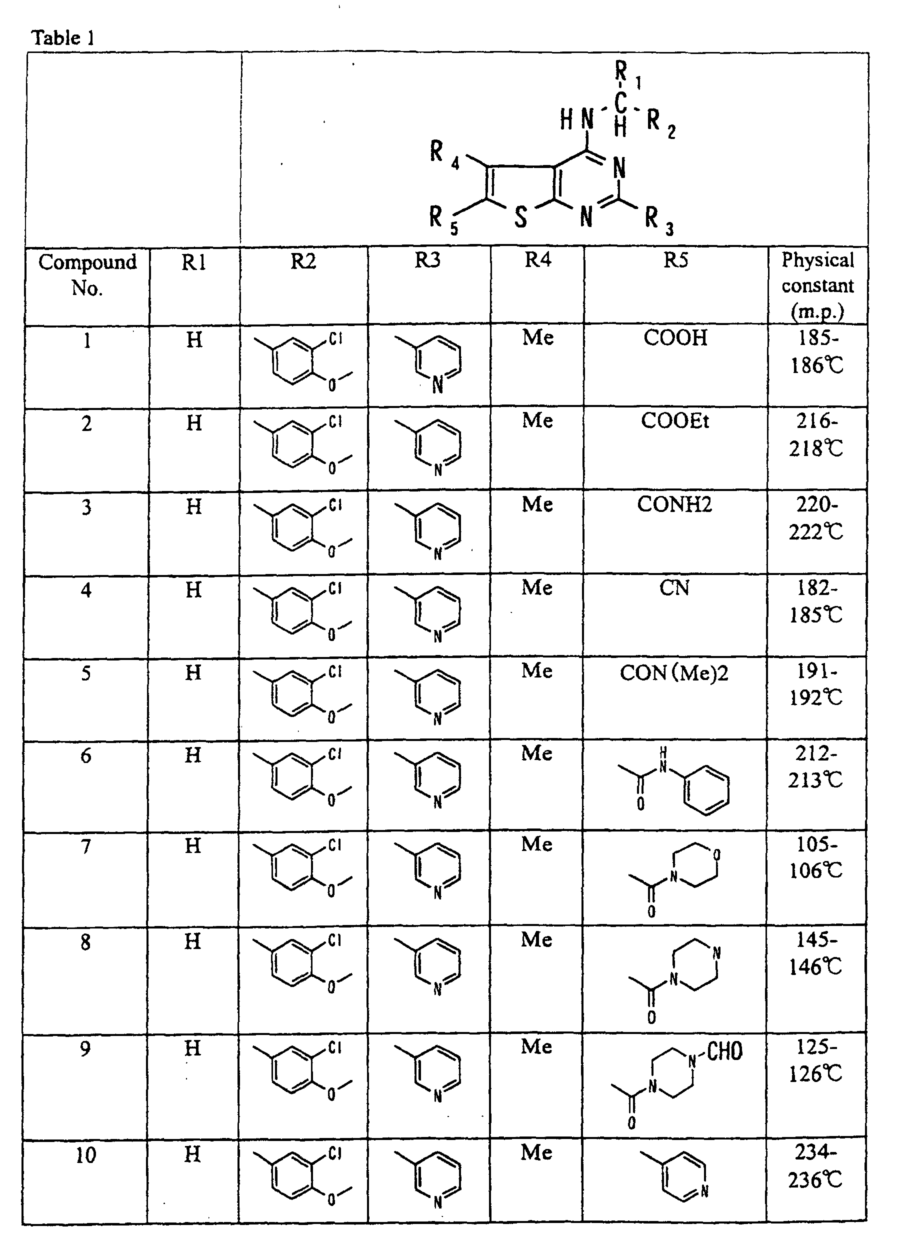

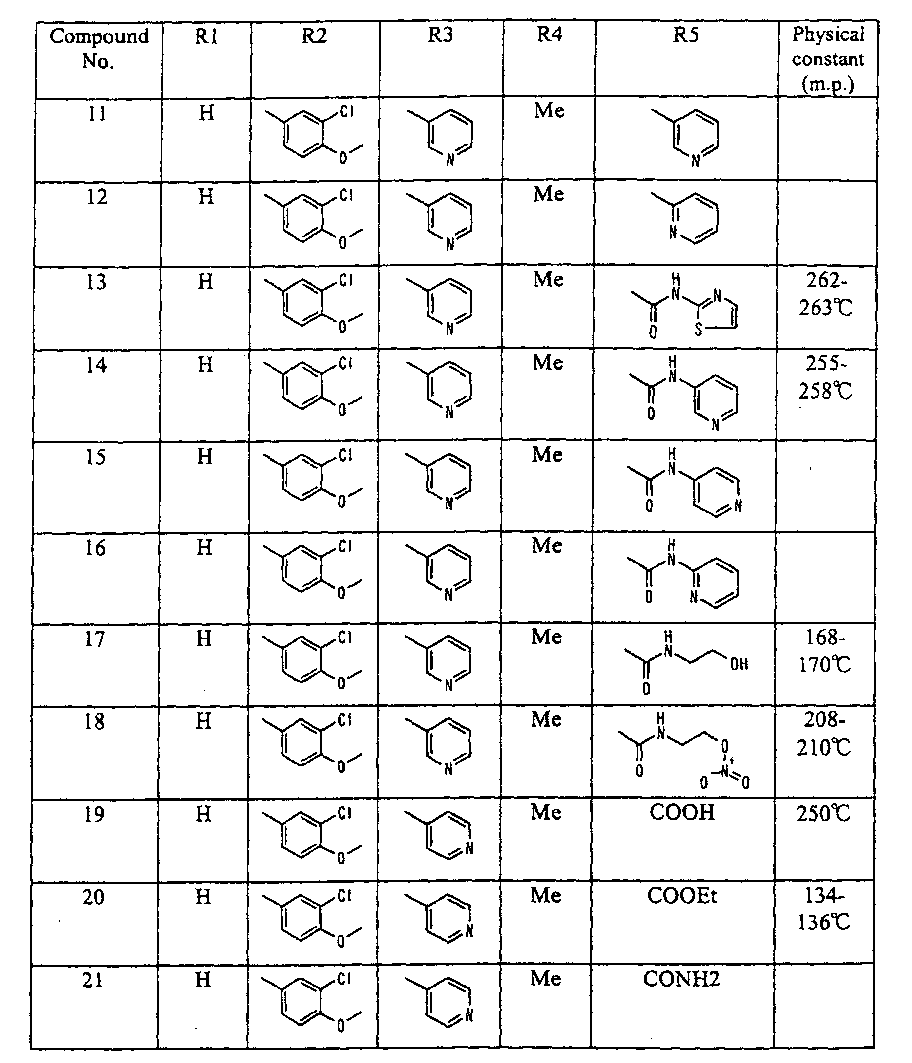

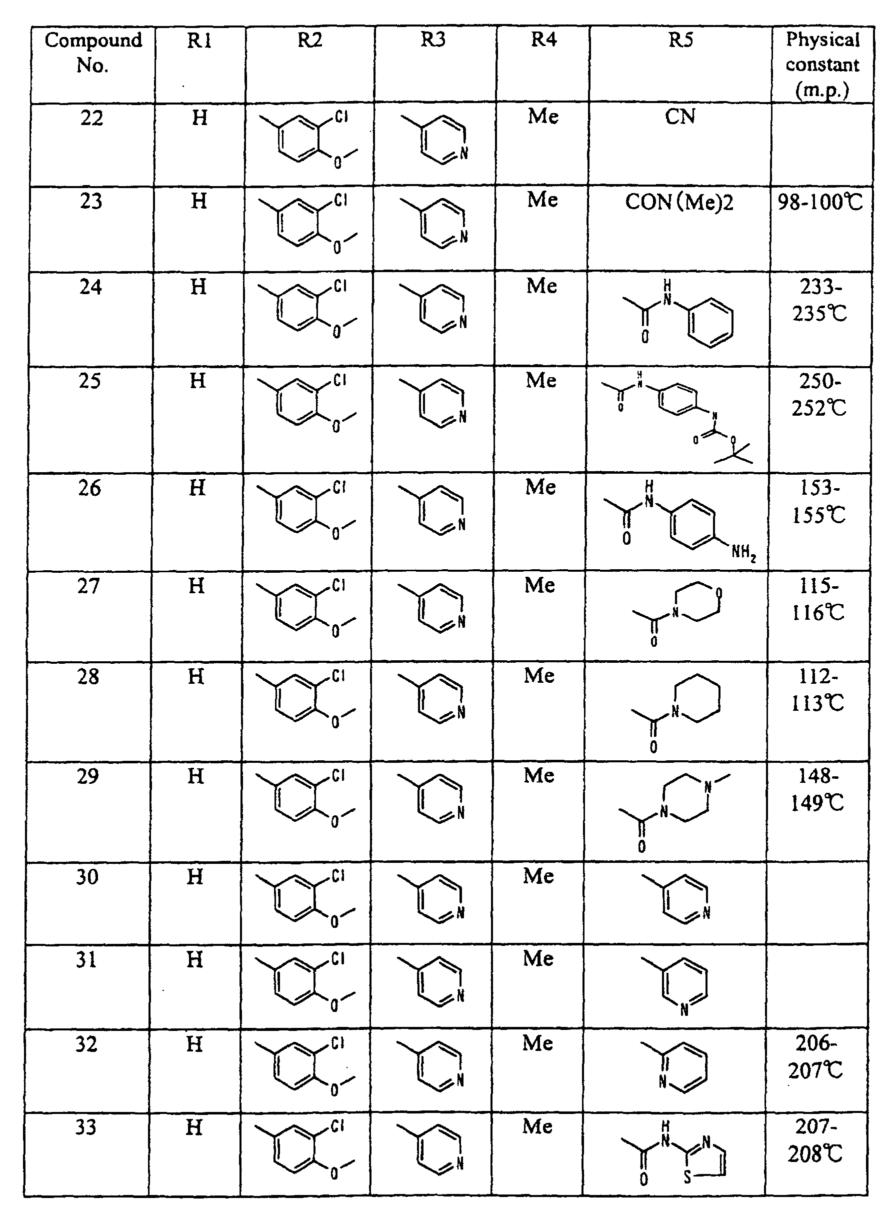

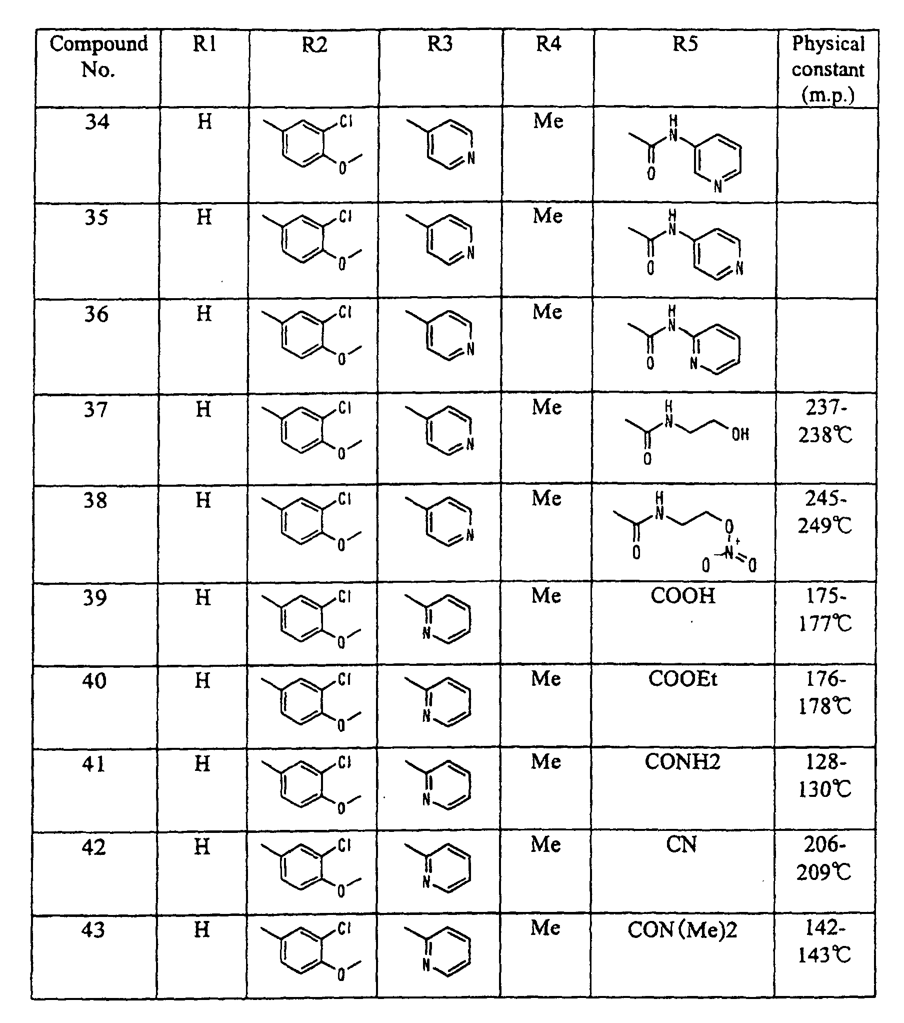

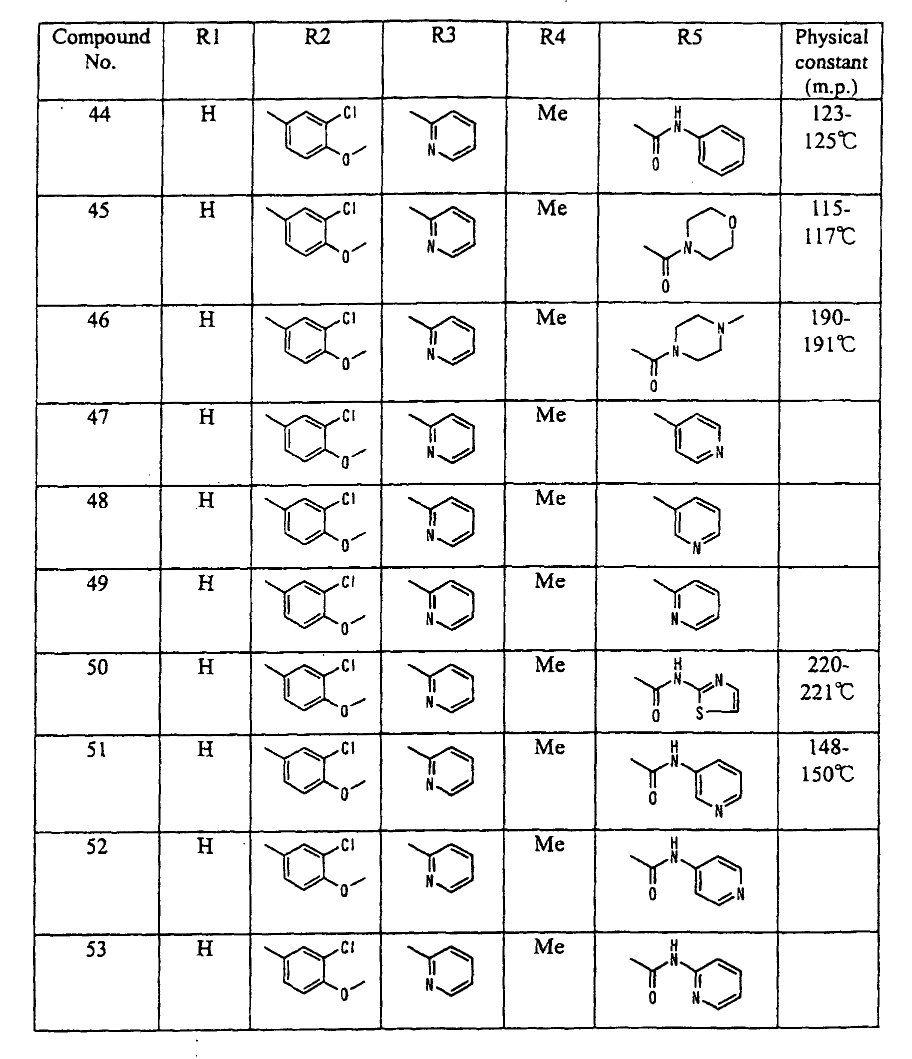

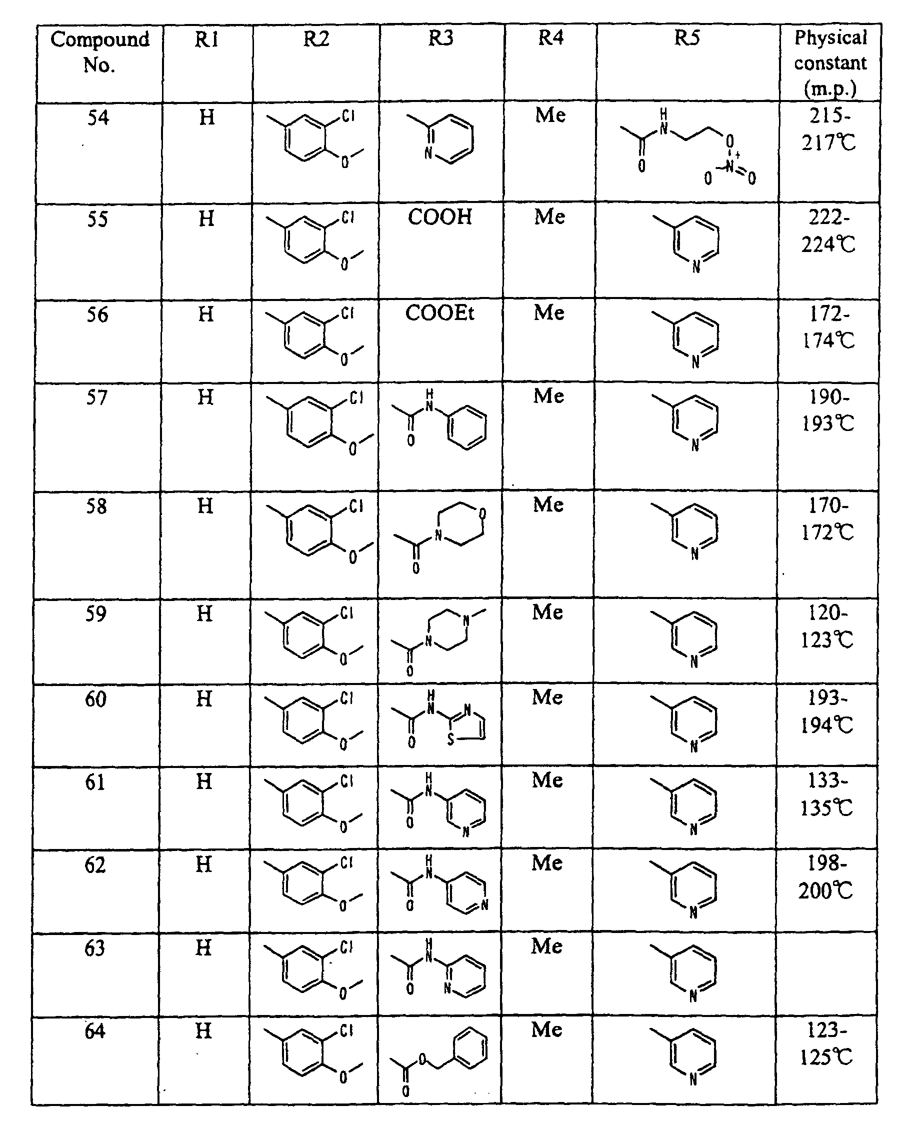

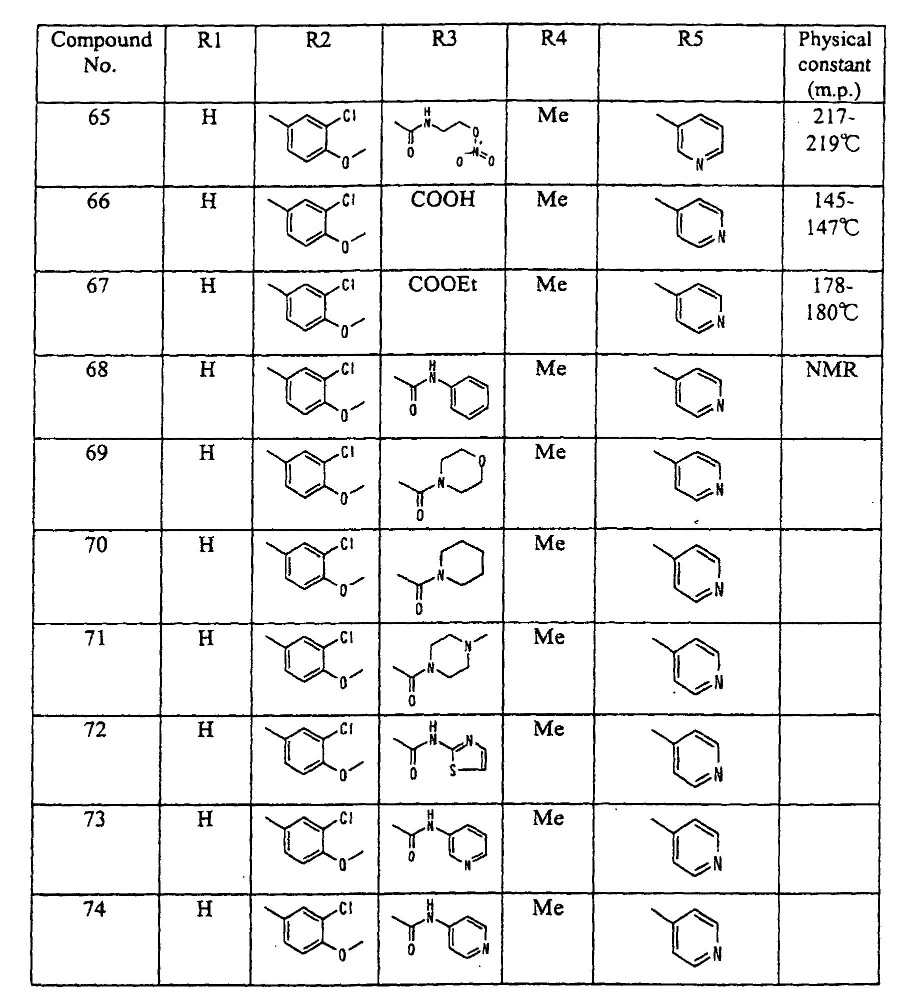

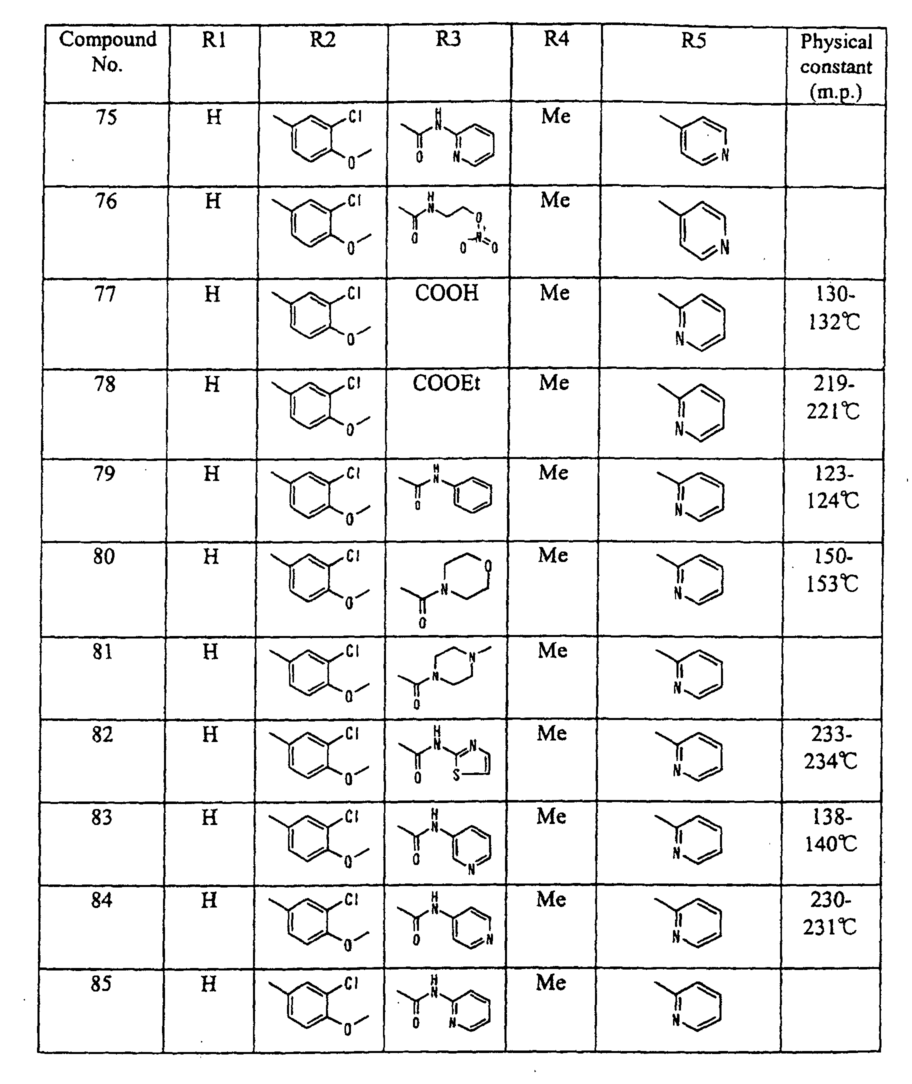

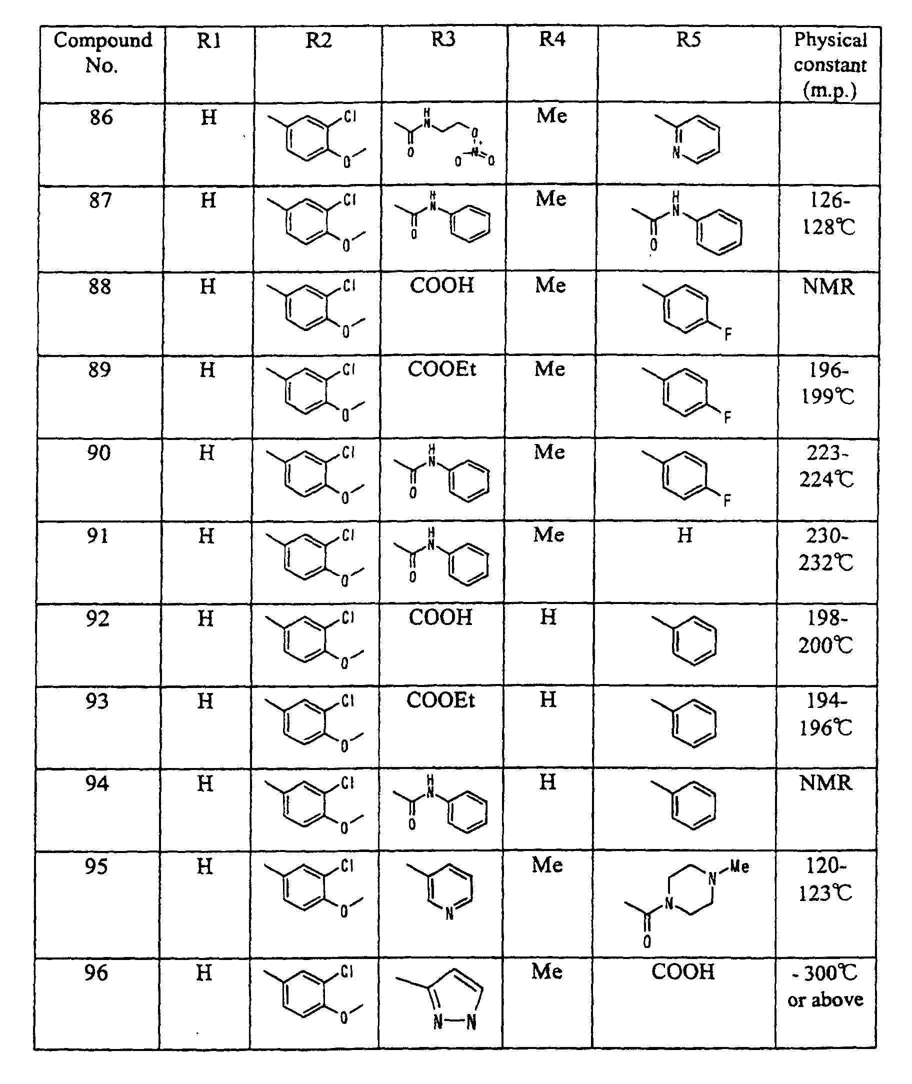

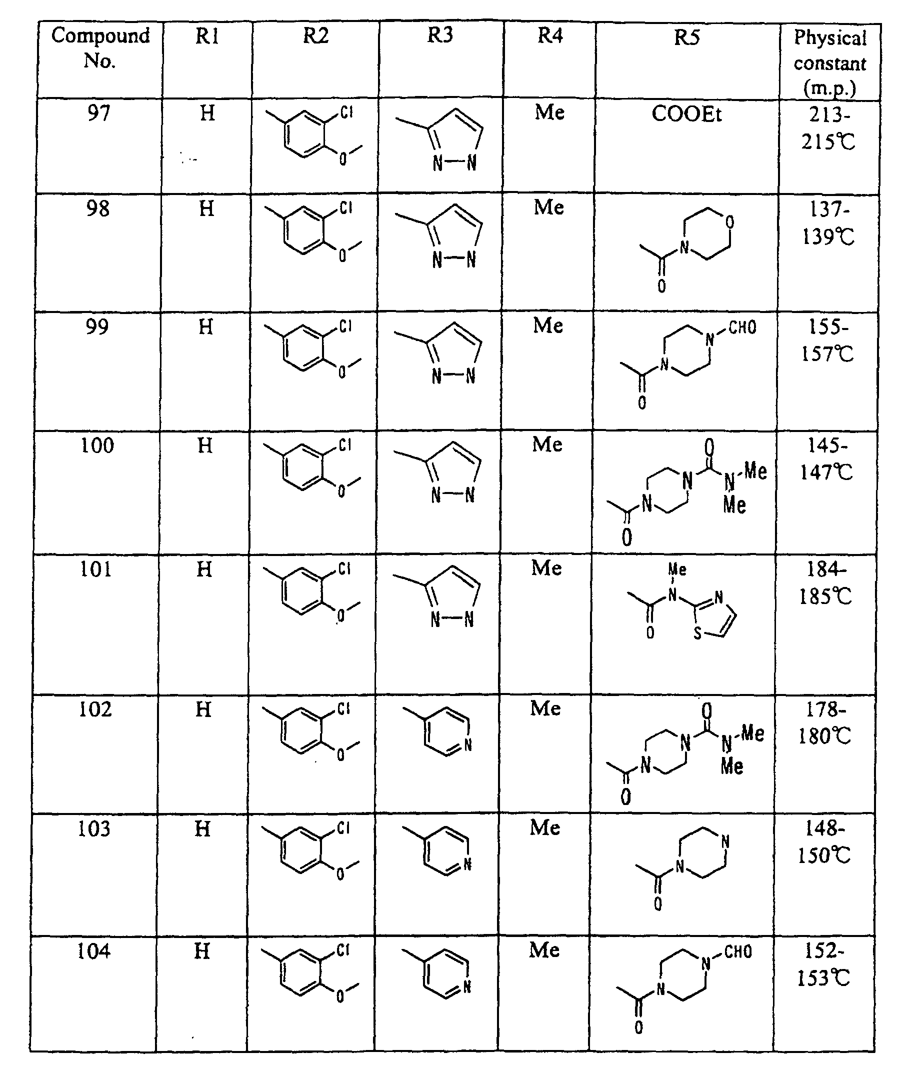

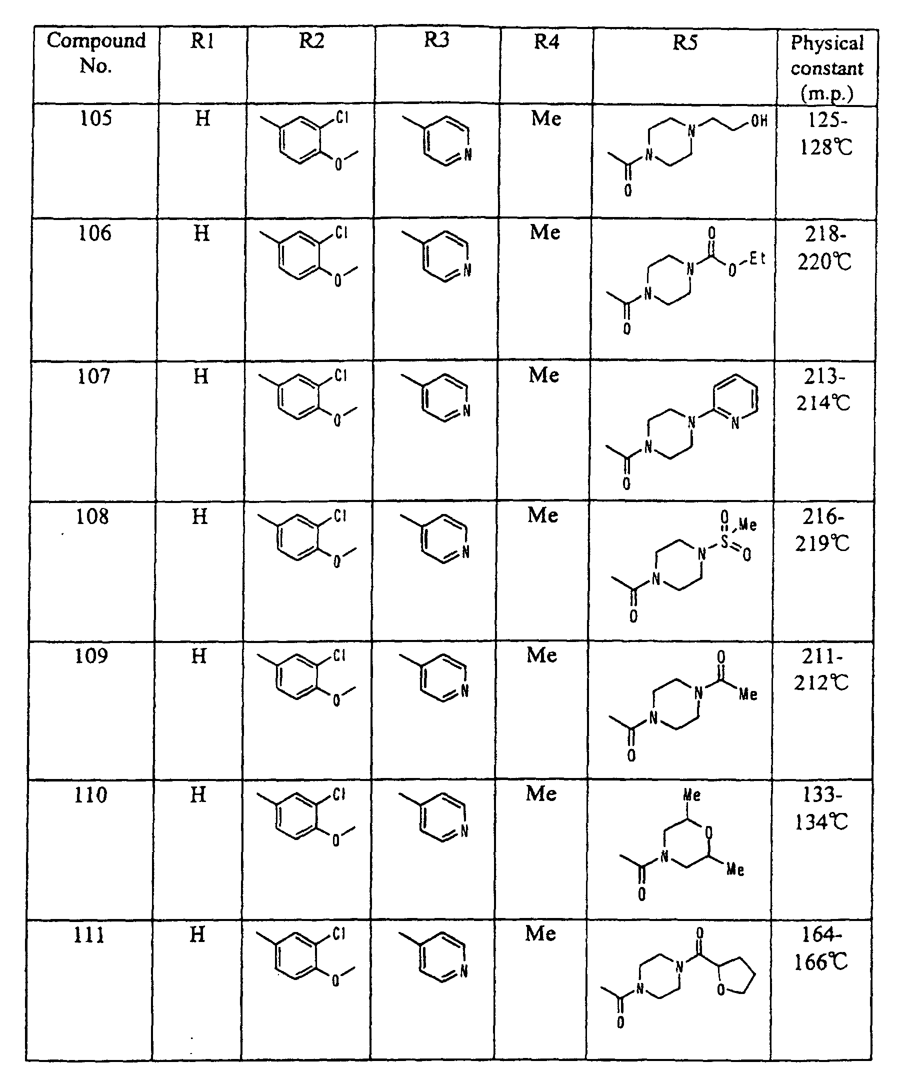

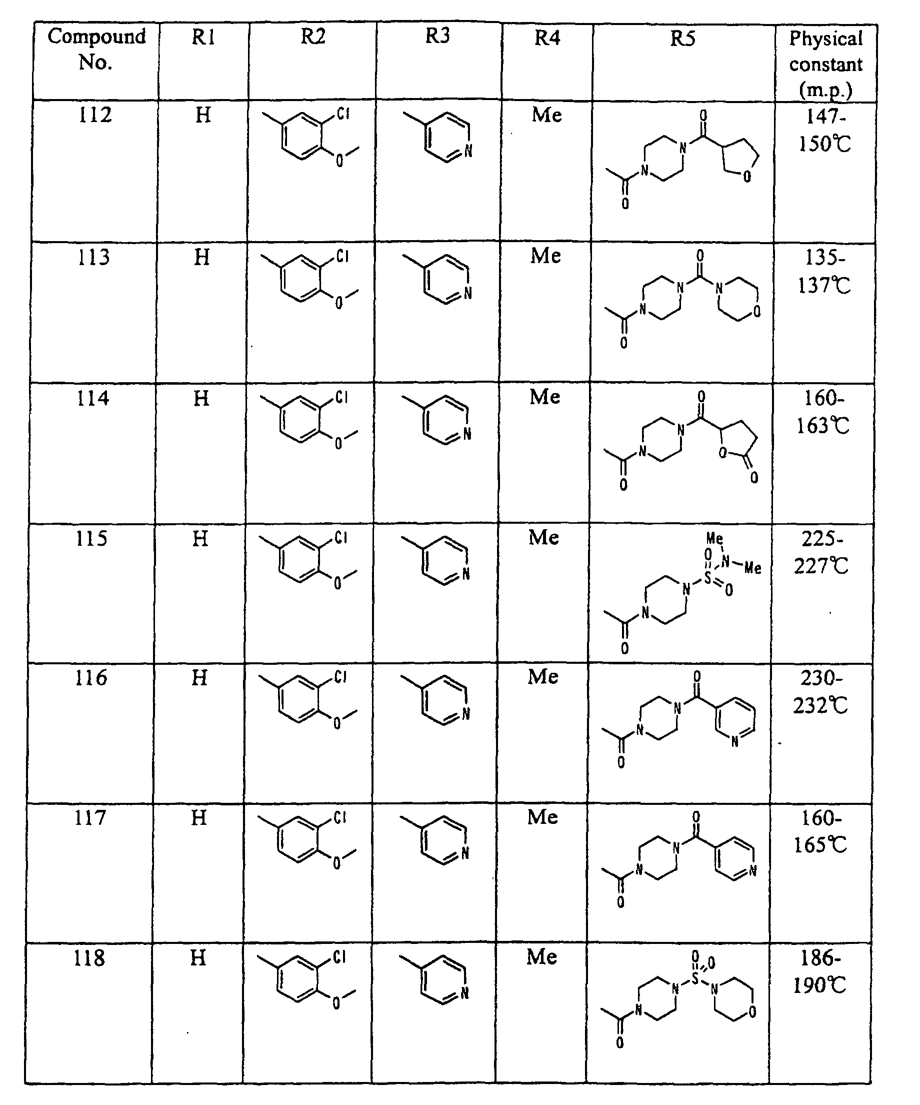

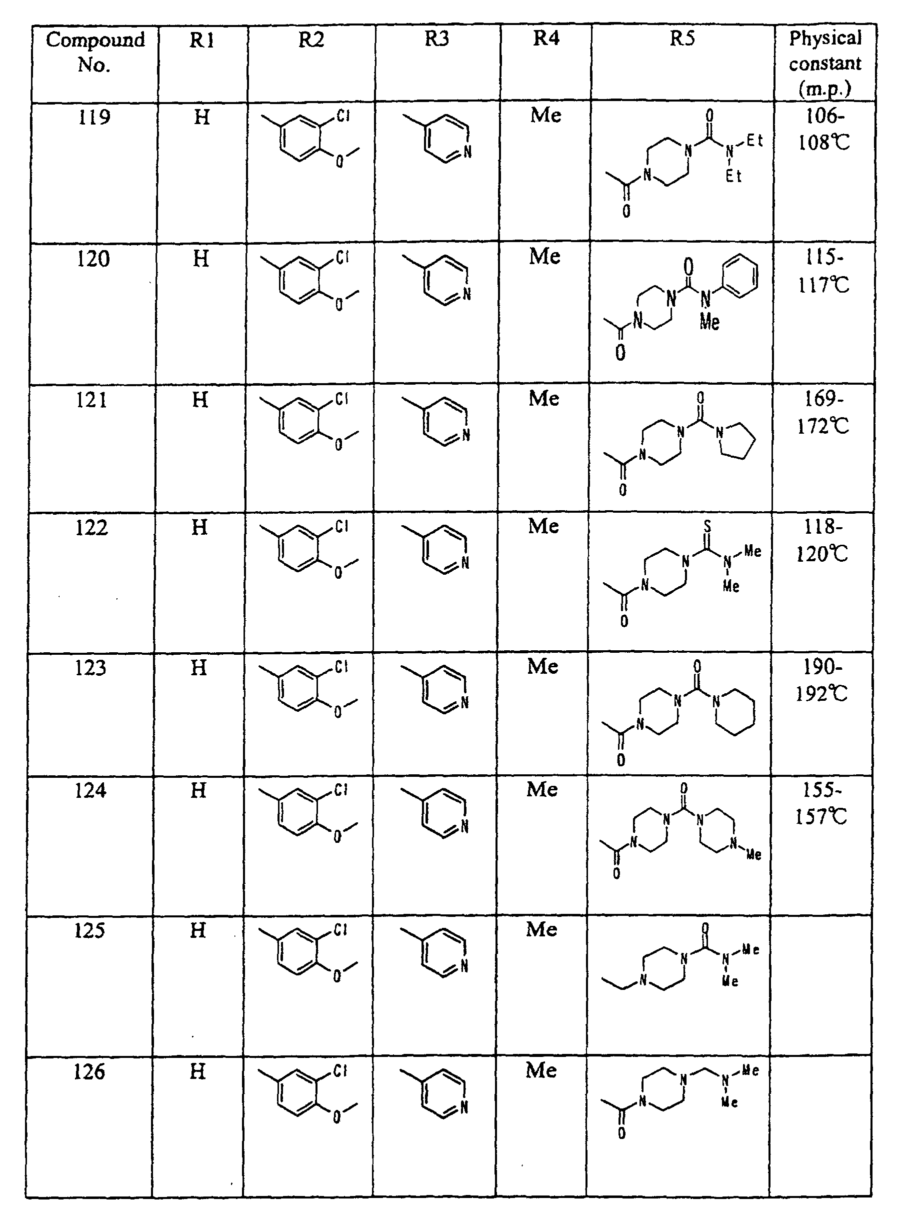

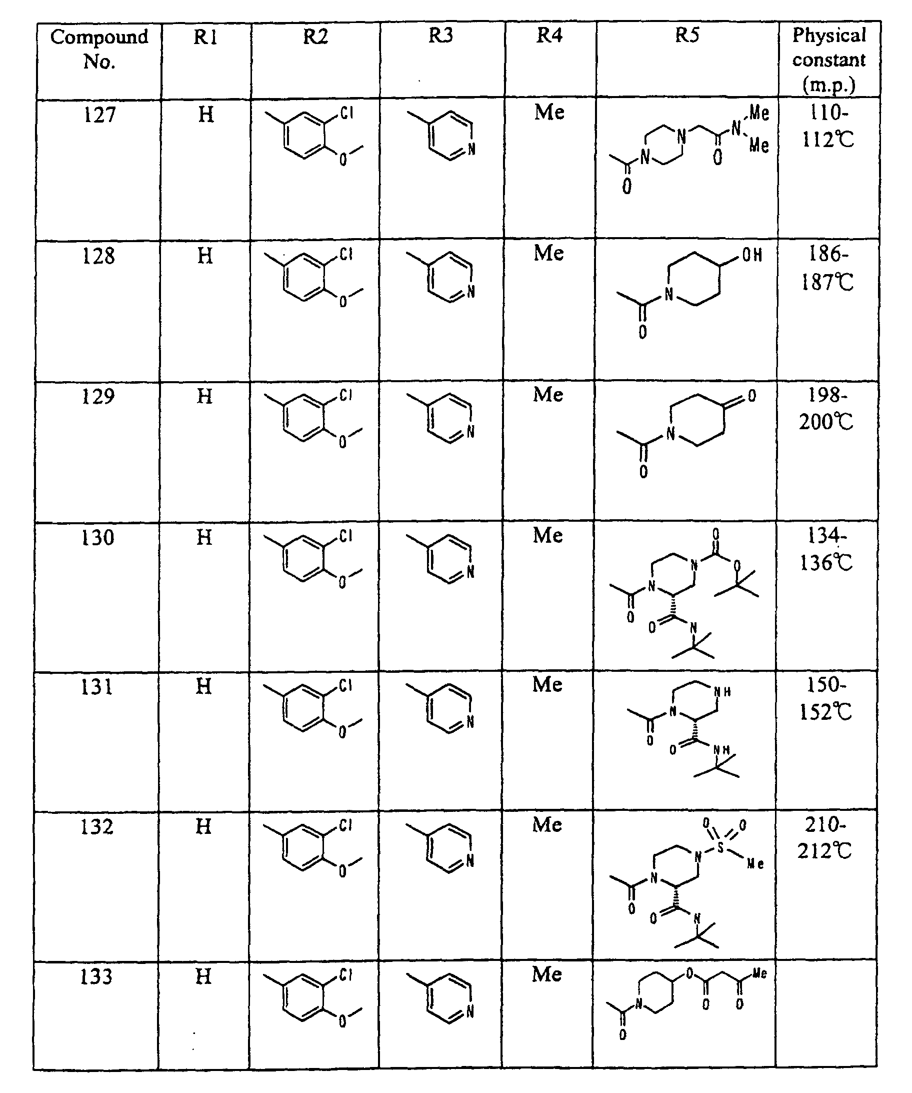

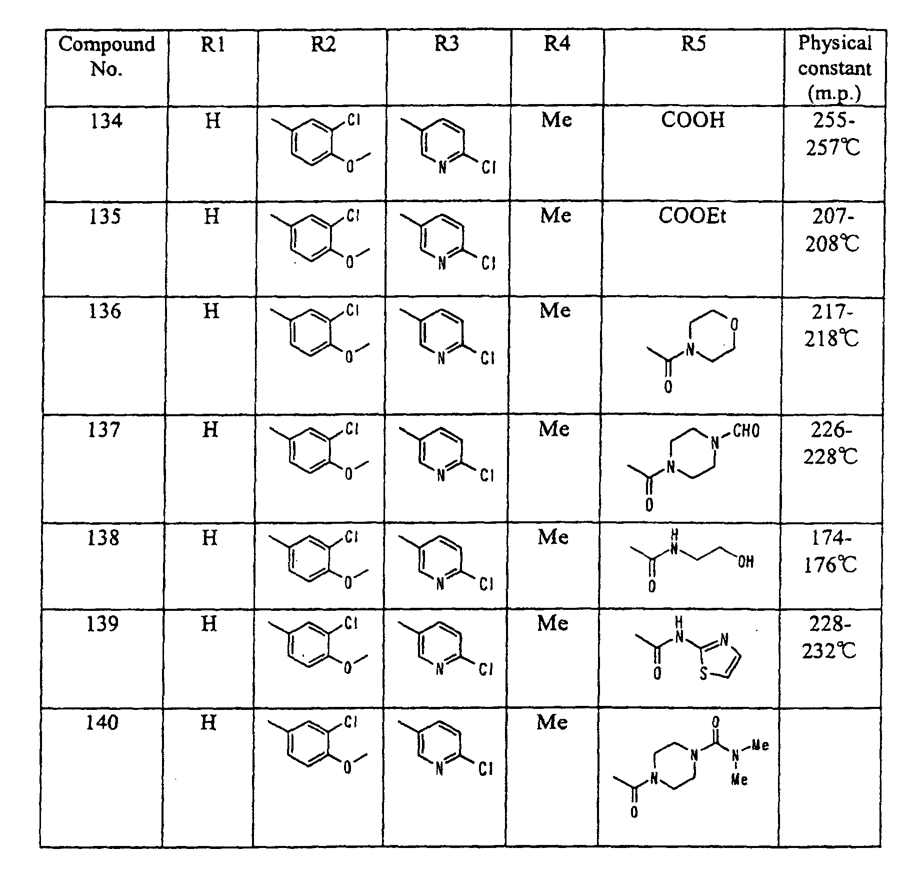

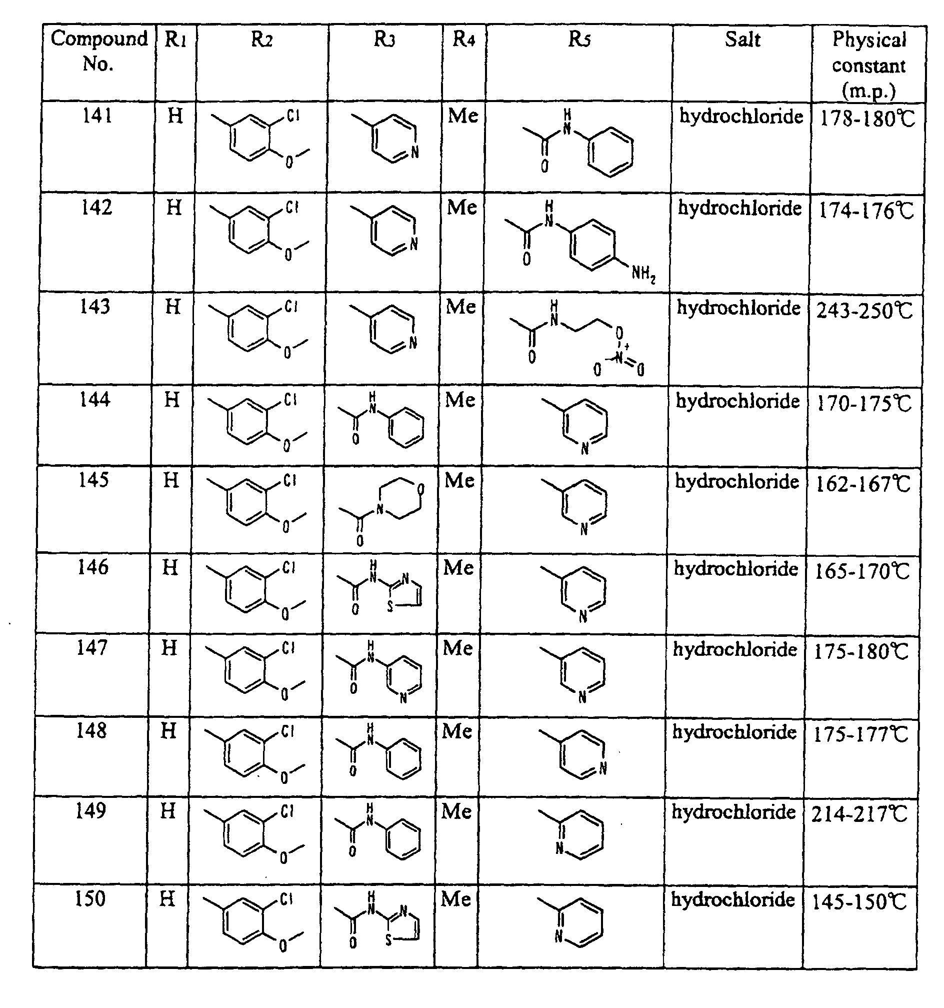

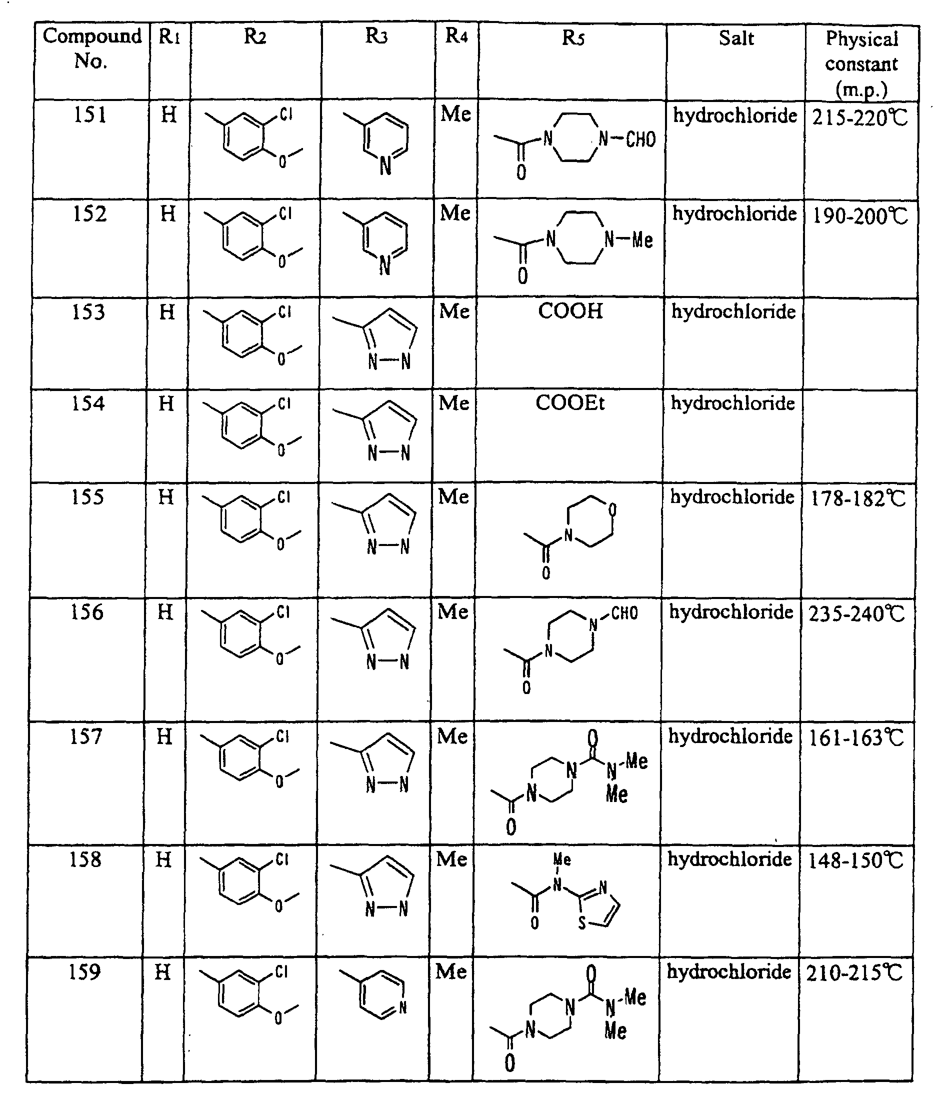

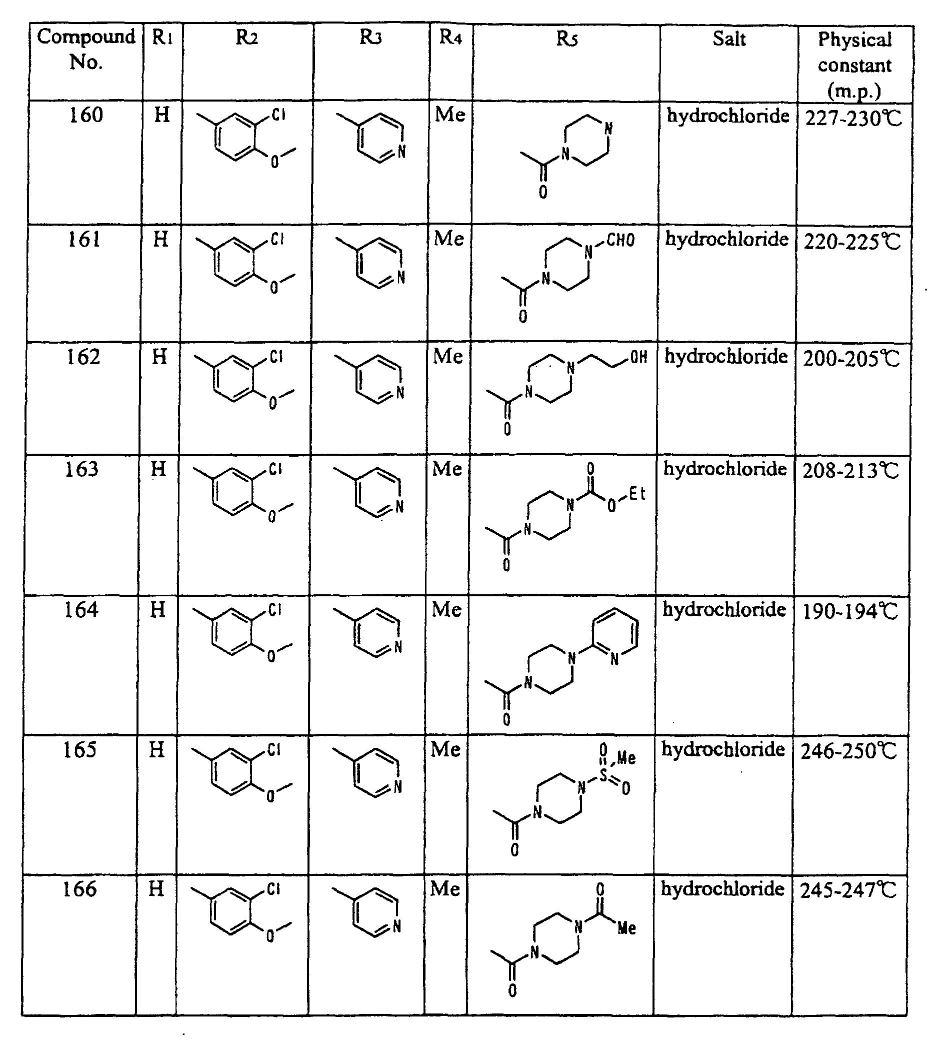

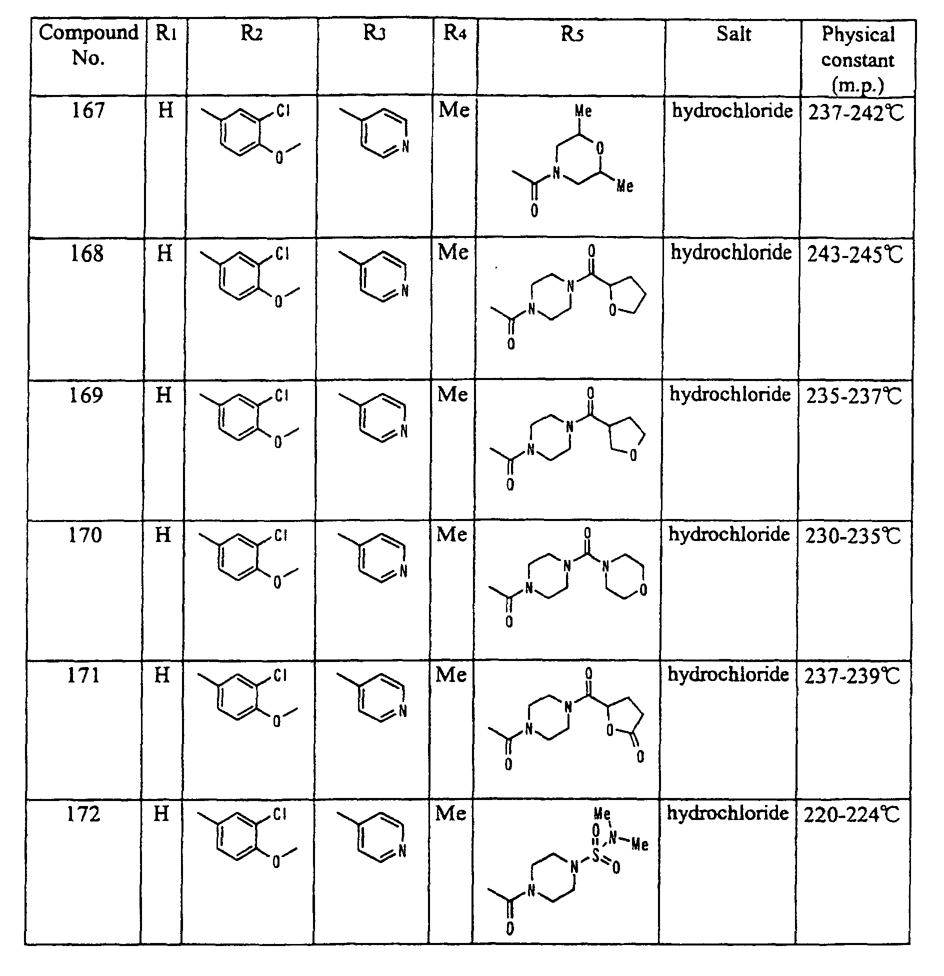

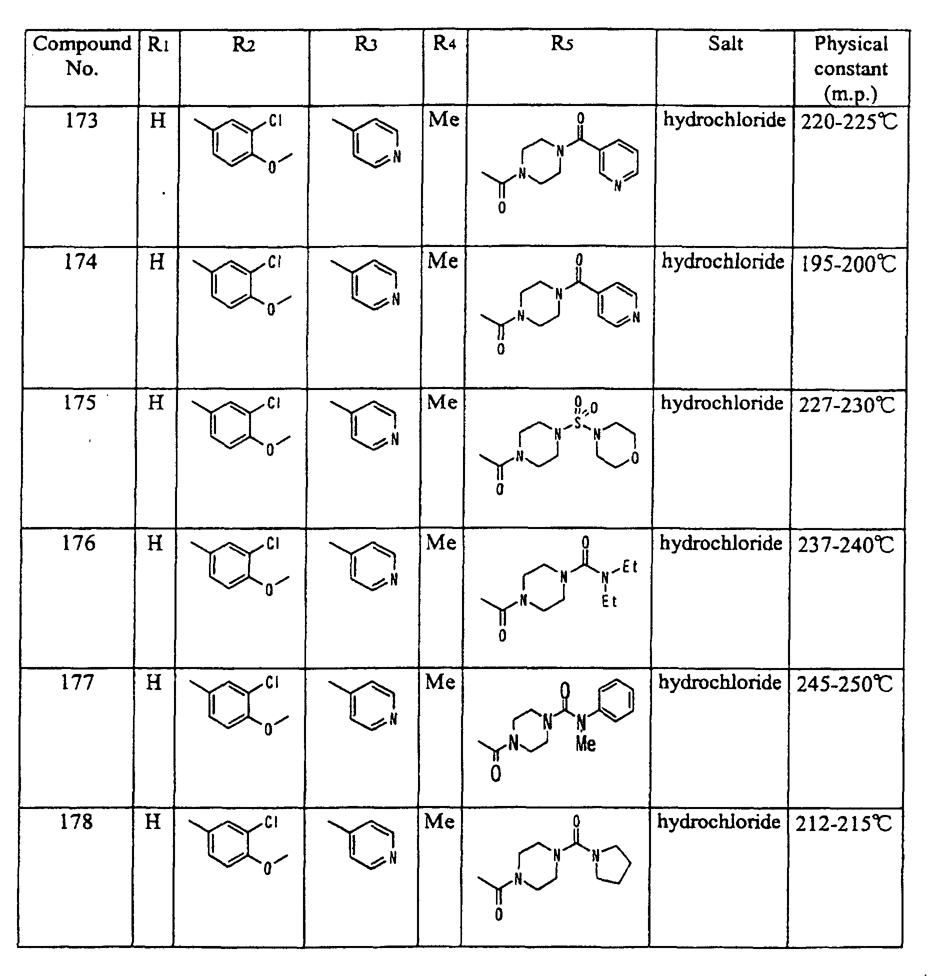

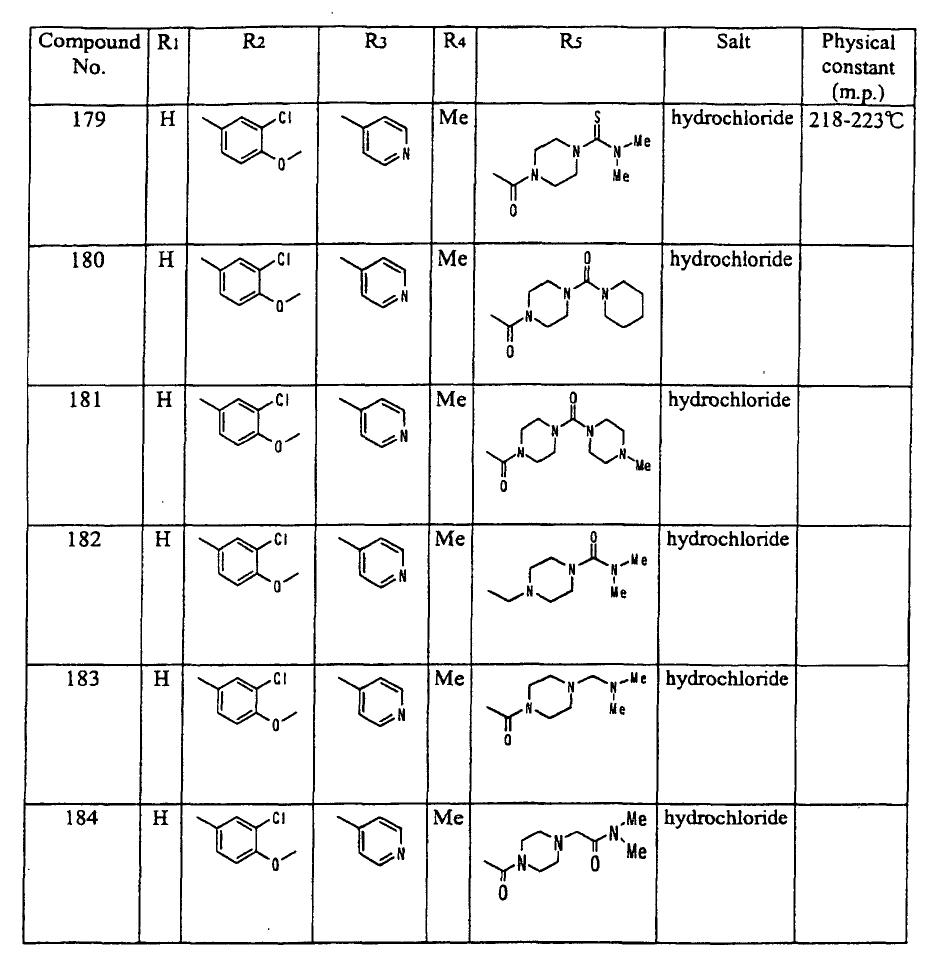

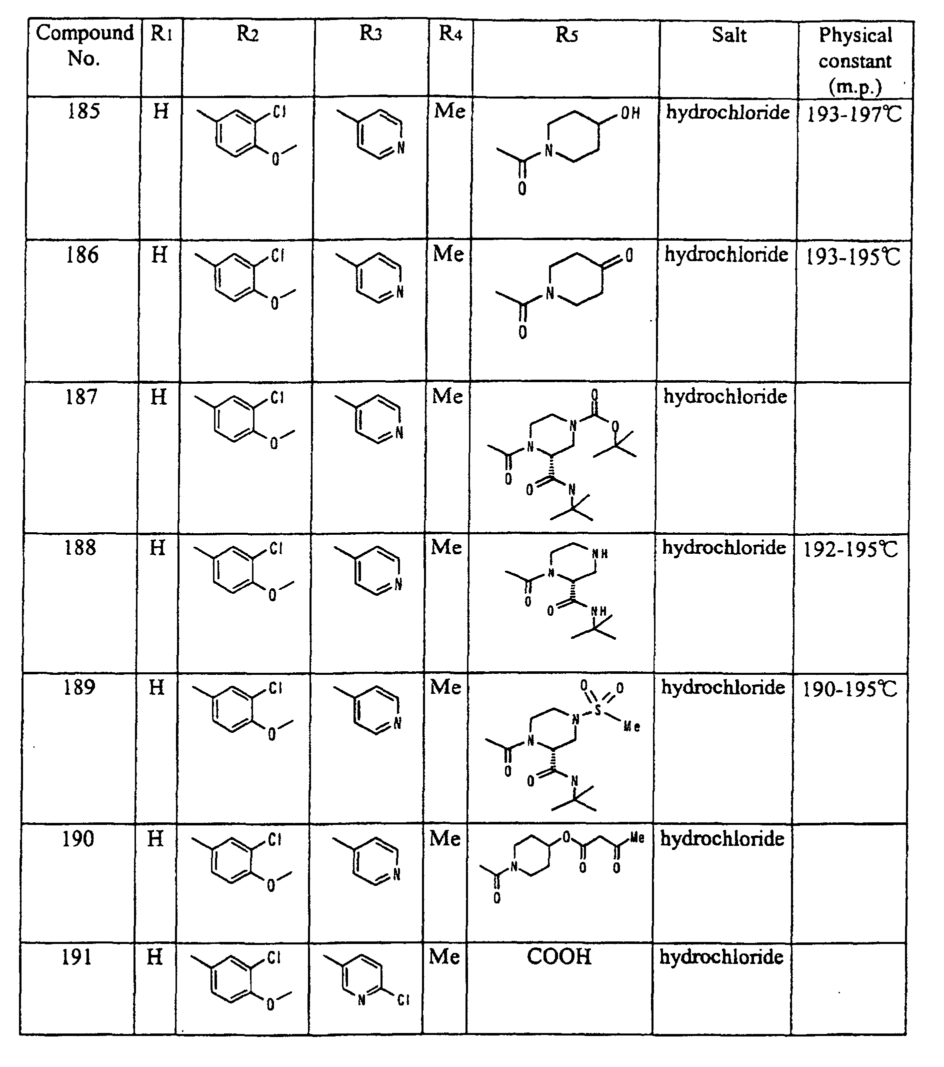

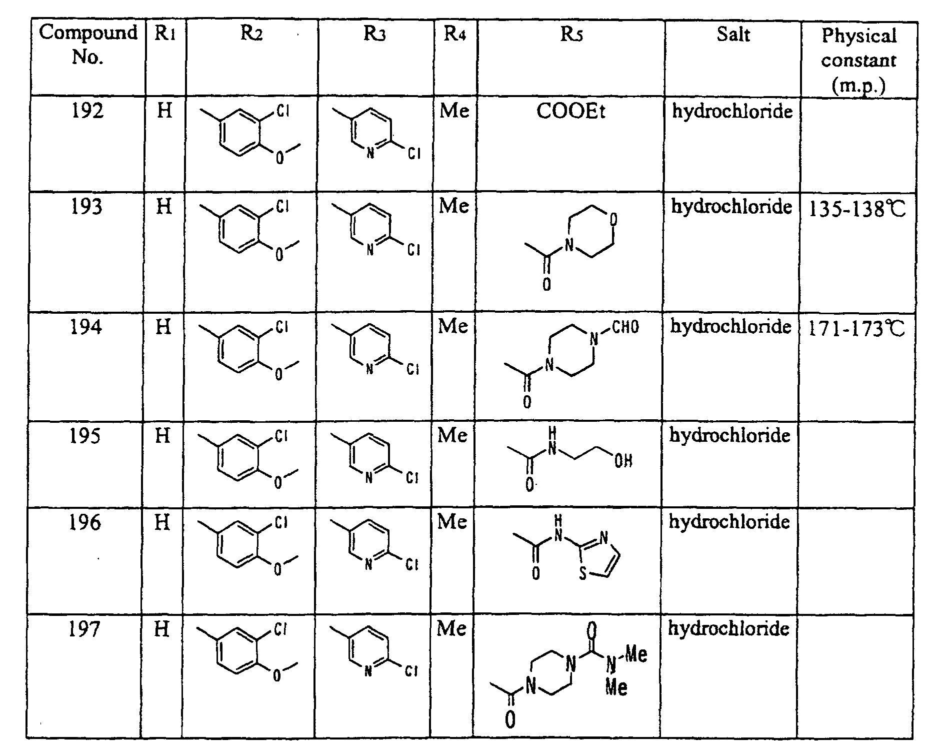

**Table 2**

| Compound No. | H-NMR (DMSO, ä ppm) |
|---|---|
| 68 | 10.3 (1H, s), 8.7 (2H, d), 7.9 (1H, t), 7.8 (2H, d), 7.7 (1H, s), 7.6 (2H, d), 7.5 (1H, d), 7.35 (2H, t), 7.2-7.0 (2H, m), 4.8 (2H, d), 3.8 (3H, s), 2.7 (3H, s) |
| 88 | 10.3 (1H, s), 7.8 (3H, m), 7.6 (3H, m), 7.5-7.3 (5H, m), 7.0-7.2 (2H, m), 4.8 (2H, d), 3.8 (3H, s), 2.6 (3H, s) |
| 94 | 10.4 (1H, s), 8.8 (1H, t), 82 (1H, s), 7.9 (2H, d), 7.7 (2H, d), 7.6-7.3 (7H, m), 7.2 (2H, t), 4.9 (2H, d), 3.8 (3H, s) |

Pharmacological activities of the compounds of the present invention are described in the following.

### Pharmacological Test Example: Phosphodiesterase Inhibiting Effect

Cyclic nucleotide phosphodiesterases from human platelets and from the heart and kidney of a dog were eluted by the concentration gradient method with 70 to 1000 mM of sodium acetate on DEAE-cellulose column chromatography (Whatman Co. Ltd., DE-52, φ 3.2 x 13 cm) according to the method of Thompson and others (Thompson W. J., et al., Advances in Cyclic Nucleotide Research: 10, 69-92, 1979), and separated into isozymes. PDE 5 (cGMP specific PDE), PDE 3 (cGMP inhibited PDE) and PDE 2 (cGMP stimulated PDE) were separated from the platelets, PDE 1 (Ca-calmodulin dependent PDE) and PDE 4 (cAMP specific PDE) were separated from the heart and the kidney respectively. The method of Thompson, et al was partially modified to measure phosphodiesterase activities: li M of [3H]-cAMP or [3H]-cGMP was decomposed with phosphodiesterase. The produced 5'-AMP or 5'-GMP was decomposed to adenosine or guanosine with a snake venom (Sigma V7000). The reaction solution was added to anion exchange resin (Bio-Rad Co. Ltd., AG1-X8). Non-adsorbed adenosine or guanosine was counted by a liquid scintillation counter. A concentration to control 50% of the enzyme activity (IC50) was calculated from concentration inhibition curves. The results are shown in Table 3.

**Table 3**

| Compound No. | PDE inhibiting activity : IC50(nM) | | | | |
|---|---|---|---|---|---|
| | PDE1 | PDE2 | PDE3 | PDE4 | PDE5 |
| 1 | 7100 | - | - | 1200 | 3.0 |
| 3 | - | - | - | | 2.3 |
| 4 | - | - | - | | 4.5 |
| 5 | >10000 | - | - | 1400 | 3.7 |
| 6 | >10000 | - | - | | 1.5 |
| 7 | >10000 | - | - | 1700 | 3.5 |
| 9 | - | - | - | | 6.1 |
| 10 | >10000 | - | - | | 3.3 |
| 13 | - | - | - | | 0.32 |
| 14 | - | - | - | | 2.1 |
| 17 | - | - | - | | 2.8 |
| 18 | - | - | - | | 2.8 |
| 19 | >10000 | - | - | 4400 | 0.74 |
| 20 | >10000 | - | - | >10000 | 3.2 |
| 23 | >10000 | - | - | 1600 | 1.6 |
| 24 | - | - | - | | 0.46 |
| 25 | - | - | - | - | 0.52 |
| 26 | - | - | - | - | 0.50 |
| 27 | >10000 | - | - | 3700 | 0.53 |
| 28 | >10000 | - | - | 1600 | 0.50 |
| 29 | >10000 | - | - | 6900 | 0.52 |
| 32 | >10000 | - | - | 240 | 0.68 |
| 33 | - | - | - | - | 0.095 |
| 38 | - | - | - | - | 0.65 |
| 44 | >10000 | - | - | - | 6.4 |
| 50 | - | - | - | - | 2.3 |
| 51 | - | - | - | - | 7.2 |
| 57 | - | - | - | - | 0.29 |
| 68 | >10000 | - | 2400 | 10000 | 0.27 |
| 87 | - | - | - | - | 0.27 |
| 90 | >10000 | - | > 10000 | 8400 | 0.43 |
| 91 | >10000 | - | >10000 | 3400 | 2.5 |
| 93 | >10000 | - | >10000 | >10000 | 2.9 |
| 94 | >10000 | - | >10000 | 5600 | 0.71 |
| Control | 2000 | 30000 | 53000 | >1000 | 14 |
| (Note) Control : Sildenafil | | | | | |

### Industrial Applicability:

The compounds of the present invention have powerful inhibiting activities, highly selective cGMP-specific PDE inhibiting effects and vasodilating effects, and are useful for the prevention and/or treatment of, for example, hypertension, heart failure, cardiac infarction, angina pectoris, arteriosclerosis, restenosis after PTCA (percutaneous transluminal coronary angioplasty), cardiac edema, pulmonary hypertension, renal failure, renal edema, hepatic edema, asthma, bronchitis, dementia, immunodeficiency, glaucoma or impotentia.

## Claims

1. A thienopyrimidine compound represented by Formula (1) 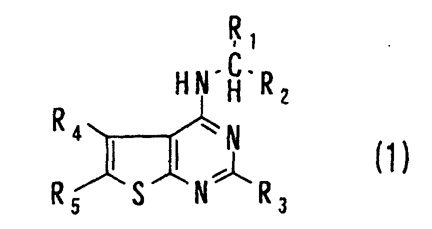 [wherein, R₁ is hydrogen or C₁₋₆ alkyl;
R₂ is C₃₋₆ cycloalkyl optionally substituted with G₁, phenyl optionally substituted with G₂, or a saturated or unsaturated heterocyclic group containing 1 to 4 heteroatoms selected from among N, O and S and optionally substituted with G₃;
R₃ is a saturated or unsaturated heterocyclic group containing 1 to 4 heteroatoms selected from among N, O and S and optionally substituted with G₃, or a group represented by Formula (CH₂)ₖC(=O)R₆ or CH=CHC(=O)R₆;
R₄ is hydrogen, C₁₋₆ alkyl, hydroxyl, C₁₋₆ alkoxy, halogen, C₁₋₆ haloalkyl, nitro or cyano;
R₅ is cyano, phenyl optionally substituted with G₂, a saturated or unsaturated heterocyclic group containing 1 to 4 heteroatoms selected from among N, O and S and optionally substituted with G₃, or a group represented by Formula (CH₂)ₖC(=O)R₆ or CH=CHC(=O)R₆;
R₆ is hydrogen, hydroxyl, C₁₋₆ alkoxy, phenoxy optionally substituted with G2, benzyloxy optionally substituted with G₂, or a group represented by Formula Nr₁r₂ or NHNr₃r₄;
r₁ and r₃ are hydrogen, or C₁₋₆ alkyl optionally substituted with hydroxyl or a ONO₂ group;
r₂ and r₄ are hydrogen, C₁₋₆ alkyl, C₃₋₈ cycloalkyl, C₁₋₆ alkoxycarbonyl, C₁₋₆ alkyl optionally substituted with a saturated or unsaturated heterocyclic group containing 1 to 4 heteroatoms selected from among N, O and S and optionally substituted with G₃, phenyl optionally substituted with G₂, benzyl optionally substituted with G₂, or a saturated or unsaturated heterocyclic group containing 1 to 4 heteroatoms selected from among N, O and S and optionally substituted with G₃;
or r₁ and r₂ may join, together with N, to form a ring shown below and optionally substituted with r₅ 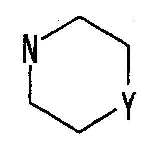 (wherein, Y is O, CH₂ or NH);
r₅ is C₁₋₆ alkyl optionally substituted with G₂, phenyl optionally substituted with G₂, benzyl optionally substituted with G₂, pyridyl optionally substituted with G₂, or -Z-Q (wherein, Z is CO, CS or SO₂, and Q is hydrogen, C₁₋₆ alkyl, C₁₋₆ alkoxy, di-C₁₋₆ alkylamino, phenyl-C₁₋₆ alkylamino, or a saturated or unsaturated heterocyclic group containing 1 to 4 heteroatoms selected from among N, O and S);
k is 0, 1 or 2;
G₁ is halogen, C₁₋₆ alkyl or C₁₋₆ alkoxy;
G₂ is halogen, C₁₋₆ alkyl, C₁₋₆ alkoxy or C₁₋₂ alkylenedioxy;
G₃ is halogen, C₁₋₆ alkyl, C₁₋₆ haloalkyl, C₁₋₆ alkoxy or C₁₋₆ alkoxycarbonyl;
a benzene ring, cycloalkyl group or heterocyclic ring may have two or more substituents of G₁, G₂, G₃ and r₅, which may be the same or different when two or more], and pharmaceutically acceptable salts thereof.

2. A compound represented by Formula (1-2) 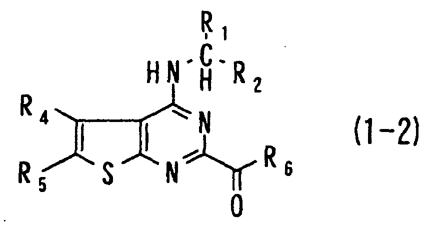 (wherein, R₁, R₂, R₄, R₅ and R₆ are as defined in Claim 1).

3. A process for the preparation of a compound of Formula (1), 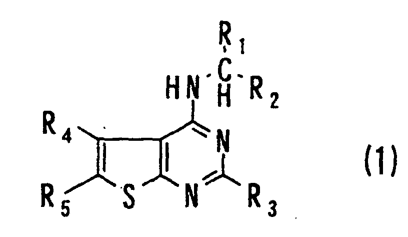 (wherein, R₁, R₂, R₃, R₄ and R₅ are as defined in Claim 1), **characterized in that** a compound of Formula (2) 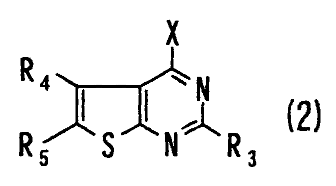 (wherein, R₃, R₄ and R₅ are as defined in Claim 1, and X is halogen), is reacted with a compound of Formula (3) 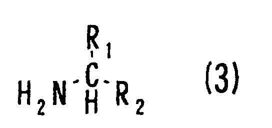 (wherein, R₁ and R₂ are as defined in Claim 1).
